# EUROPÄISCHE PATENTSCHRIFT

(11) **EP 1 963 299 B1**
(45) Veröffentlichungstag und Bekanntmachung des Hinweises auf die Patenterteilung: **18.01.2012**
(21) Anmeldenummer: 06829183.0
(22) Anmeldetag: 29.11.2006
(51) Int. Cl.: C07D 311/80, C07D 491/04

(54) **OXAPHENANTHREN-DERIVATE**
OXAPHENANTHRENE DERIVATIVES
DERIVES OXAPHENANTHRENE

(30) Priorität: 22.12.2005 DE 102005062098
(43) Veröffentlichungstag der Anmeldung: 03.09.2008
(73) Patentinhaber: Merck Patent GmbH, 64293 Darmstadt (DE)
(72) Erfinder: TAUGERBECK, Andreas, 64285 Darmstadt (DE); PAULUTH, Detlef, 64372 Ober-Ramstadt (DE); KRETSCHMER, Beate, 61462 Koenigstein (DE); WURZIGER, Hanns, 64291 Darmstadt (DE); JANSEN, Axel, 64293 Darmstadt (DE); MAILLARD, David, 64285 Darmstadt (DE)
(86) Internationale Anmeldenummer: PCT/EP2006/011457
(87) Internationale Veröffentlichungsnummer: WO 2007/079830

(56) Entgegenhaltungen:
- DE-A1-102004 004 228
- ROG, D.J. ET AL.: "Randomized, controlled trial of cannabis-based medicine in central pain in multiple sclerosis" NEUROLOGY, Bd. 65, September 2005 (2005-09), Seiten 812-819, XP002423046

## Beschreibung

Die Erfindung betrifft Verbindungen der Formel I 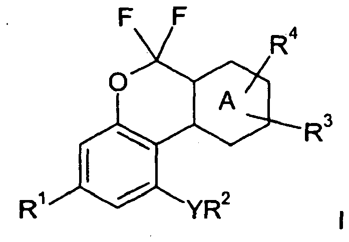 worin
- A: einen gesättigten, ungesättigten carbocyclischen oder heterocyclischen Rest mit 6 Ringgliedern, worin bei gesättigten Resten eine oder mehrere CH₂-Gruppen durch CO ersetzt sein können,
- R¹, R², R³, R⁴: unabhängig voneinander H, Hal, verzweigtes oder unverzweigtes Alkyl mit 1 bis 12 C-Atomen, worin auch eine oder mehrere H-Atome durch F, eine oder mehrere CH₂-Gruppen durch -C=C-,-C≡C-, C=O, -O-, -S-, -NH-, -NR¹- oder substituiertes oder unsubstituiertes Phenylen und eine oder mehrere CH₃-Gruppen durch 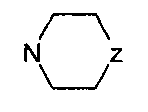 ersetzt sein können, worin z, CHR⁵, S, O, NR⁵ oder NR⁵ bedeutet,
Hal Fluor, Chlor, Brom oder Iod,
Y eine Einfachbindung, O, S oder NR⁵;
R⁵, H oder Alkyl

### bedeuten,

sowie deren physiologisch unbedenkliche Salze und Solvate, Steroisomere und deren Mischungen in allen Verhältnissen.

Verbindungen der Formel I können aufgrund asymmetrisch substituierter Kohlenstoffatome als Stereoisomere vorliegen. Gegenstand der Erfindung sind daher sämtliche Isomere, sowohl in reiner Form, als Racemat und auch als Mischung von Diastereomeren oder Enantiomeren.

Der Erfindung lag die Aufgabe zugrunde, Inhibitoren von Cannabinoidrezeptoren (CB-Rezeptoren) aufzufinden und zu optimieren. Die vorliegende Erfindung bezieht sich auf neue therapeutische und/oder prophylaktische Verwendungen von Verbindungen, die CB-Rezeptoren inhibieren, und auf pharmazeutische Zubereitungen, die eine oder mehrere dieser Verbindungen als eine aktive Komponente enthalten. Die in dieser Erfindung genannten Verbindungen zeigen CB-Rezeptor-inhibierende Eigenschaften und können von herausragender Bedeutung für neue medizinische Anwendungen sein.

Cannabinoide kommen in der indianischen Hanfpflanze *Cannabis Sativa L.* vor und werden seit Jahrhunderten zur medizinischen Anwendung genutzt (Mechoulam, R.; Feigenbaum, J. J. Prog. Med. Chem. 1987, 24, 159). Jedoch brachte die Forschung auf dem Gebiet der Cannabinoide erst in der letzten Dekade entscheidende Informationen über CB-Rezeptoren zu Tage. Die Entdeckung und Klonierung zweier Cannabinoid-Rezeptorsubtypen (CB1 und CB2) regte die Suche nach neuen Cannabinoid-Rezeptor-Antagonisten an (Munro, S.; Thomas, K. L.; Abu- Shaar, M. Nature 1993, 365, 61. Matsuda, L. A.; Bonner, T. I. Cannabinoid Receptors, Pertwee, R. G. Ed. 1995, 117, Academic Press, London). Zudem begann sich die pharmazeutische Industrie für die Entwicklung von cannabinoiden Medikamenten zur Behandlung von - mit Störungen des Cannabinoid-Systems einhergehenden - Krankheiten zu interessieren. Die weite Verbreitung des CB1-Rezeptors im Hirn, in Kombination mit der streng peripheren Lokalisation des CB2-Rezeptors, machte den CB1- Rezeptor zu einem sehr interessanten molekularen Target für die ZNS-gerichtete Wirkstoffentwicklung. Insbesondere weisen die erfindungsgemäßen Verbindungen CB1- Rezeptor-Aktivität auf und sind für die Behandlung psychiatrischer Krankheiten wie Psychosen, Angststörungen, Depression, Aprosexie, Gedächtnisstörungen, kognitive Störungen, Appetitlosigkeit, Obesitas, Sucht, Drogenabhängigkeit und neurologischen Störungen wie neurodegenerative Prozesse, Demenz, Dystonie, Muskelspasmen, Tremor, Epilepsie, Multiple Sklerose, traumatische Himverletzungen, Schlaganfall, Parkinson, Alzheimer, Huntington's Disease, Tourette-Syndrom, cerebrale Ischämie, cerebralem Apoplex, craniocerebralem Trauma, Rückenmarksverletzungen, neuroinflammatorische Krankheiten, cerebrale Arteriosklerose, virale Encephalitis, Krankheiten, die mit einer Demyelinisierung einhergehen, sowie zur Behandlung von Schmerzkrankheiten einschließlich neuropathischer Schmerzkrankheiten, und anderen Krankheiten, bei denen cannabinoide Neurotransmission eine Rolle spielt, einschließlich septischer Schock, grüner Star, Krebs, Diabetes, Erbrechen, Übelkeit, Asthma, Atemwegserkrankungen, gastrointestinale Erkrankungen, Magengeschwür, Diarrhoe und kardiovaskuläre Krankheiten einsetzbar.

Es wurde nun überraschend gefunden, dass THC-Derivate (Δ⁹-Tetrahydrocannabinol-Derivate), bei denen die geminalen Methylgruppen an C9 durch Fluor ersetzt sind; verbesserte pharmakologische Wirkungen entfalten, ohne jedoch ausgeprägt psychoaktiv zu sein. Bisher waren ähnliche Verbindungen nur für ihre flüssigkristallinen Eigenschaften bekannt (JP2005120073; DE102004004228).

Die Verbindungen können weiterhin auf Muscarinische Rezeptoren wirken. Es wurde zudem gefunden, dass die Verbindungen der Formel I und ihre Salze, die wichtige Zwischenprodukte zur Herstellung von Arzneimitteln darstellen - insbesondere von solchen, die beispielsweise auf das Zentralnervensystem wirken - durch Umsetzung von Verbindungen der Formel II oder deren Salzen mit Verbindungen der Formel III oder deren Salzen erhalten werden können.

Vor- und nachstehend haben die Reste R¹ bis R⁵, Ring A, Y und Hal die bei Formel I angegebenen Bedeutungen, falls nicht ausdrücklich etwas anderes angegeben ist.

Der Rest Alkyl bedeutet vorzugsweise unverzweigtes oder verzweigtes Alkyl und hat 1 bis 12, vorzugsweise 1 bis 8 C-Atome. Alkyl bedeutet daher insbesondere z.B. Methyl, Ethyl, n-Propyl, Isopropyl, n-Butyl, sek.-Butyl oder tert.-Butyl, ferner auch Pentyl, 1-, 2- oder 3-Methylbutyl, 1,1-, 1,2- oder 2,2-Dimethylpropyl, 1-Ethylpropyl, Hexyl, 1-, 2-, 3- oder 4-Methylpentyl, 1,1-, 1,2-, 1,3-, 2,2-, 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl. Bevorzugt sind auch solche Alkyl Reste worin eine CH₂-Gruppe durch ein O-oder S-Atom oder durch eine CH=CH-Gruppe oder mindestens ein H-Atom durch F ersetzt sein kann. Bevorzugt bedeutet der Rest Alkyl einen Rest der folgenden Teilformel : C(CH₃)₂-Alk, wobei Alk einen vorzugsweise geradekettiges Alkylrests mit 1 bis 7 C-Atomen bedeutet.

Der Ring A bedeutet vorzugsweise einen gesättigten oder ungesättigten carbocyclischen oder heterocyclischen Rest mit 6 Ringgliedern, wobei im Falle eines heterocyclischen Restes 1 bis 4 Heteroatome, ausgewählt unter N, S und O, vorliegen können. Der Ring A bedeutet insbesondere z.B. Cyclohexan, worin auch eine CH₂-Gruppe durch CO ersetzt sein kann, Cyclohexen oder Piperidin.

Hal bedeutet Fluor, Chlor, Brom oder Iod, insbesondere Fluor.
R¹ R², R³, R⁴ bedeute H, Hal unverzweigtes oder verzweigtes Alkyl und hat 1 bis 12, worin auch eine oder mehrere H-Atome durch F, eine oder mehrere CH₂-Gruppen durch -C=C-, -C≡C-, CO, -O-, -S-, -NH, -NR¹- oder Phenylen und eine oder mehrere CH₃-Gruppen durch vorzugsweise 3 bis 10 C-Atome und insbesondere 5 bis 9 C-Atome, 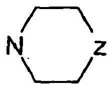 ersetzt sein können, worin
z CH₂, S, O, NR¹ oder NH bedeutet.
R⁵ bedeutet H oder Alkyl
Y bedeutet eine Einfachbindung O, S oder NR⁵, wobei R⁵ die oben genannte Bedeutung aufweist. Besonders bevorzugt bedeutet Y O.

Ganz besonders bevorzugt sind die folgenden erfindungsgemäßen Verbindungen I1 bis 139 und Ia: 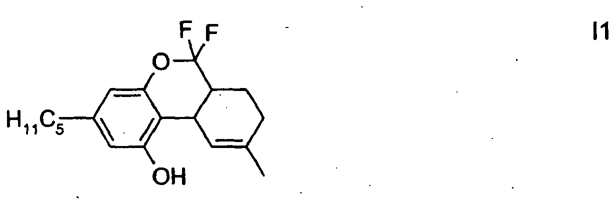 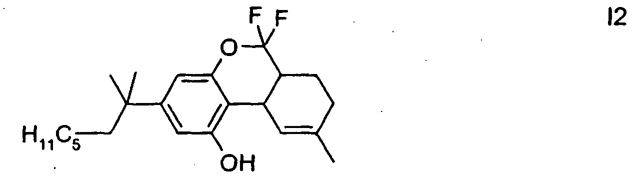 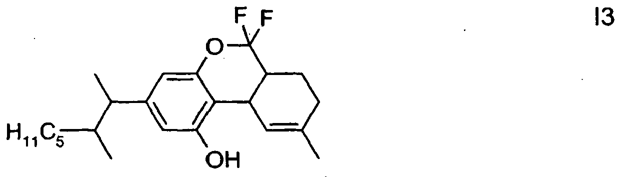 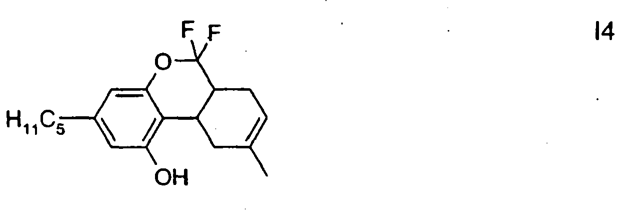 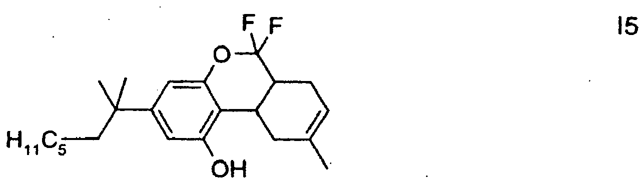 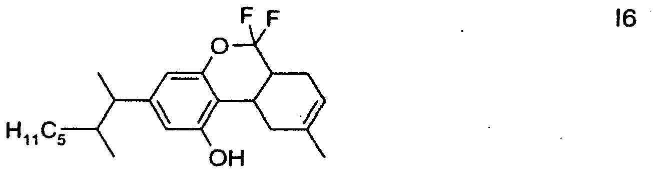 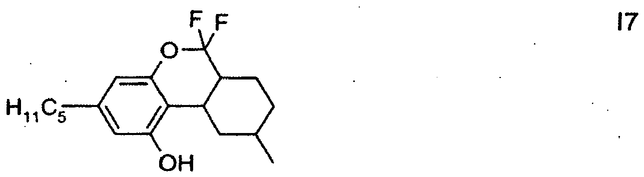 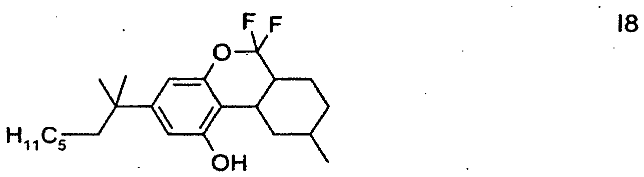 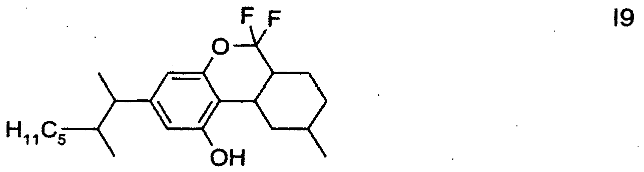 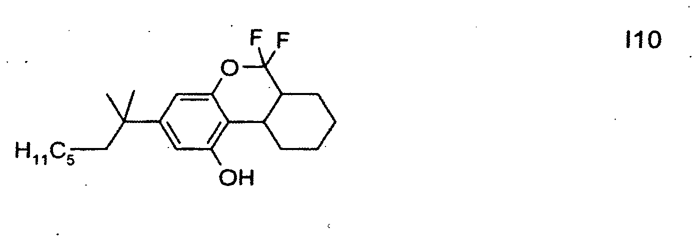 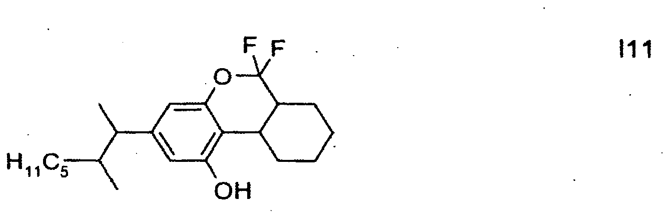 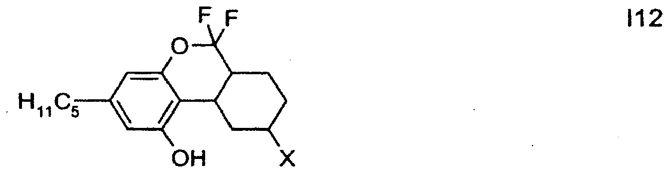 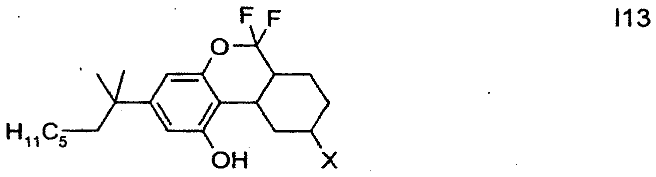 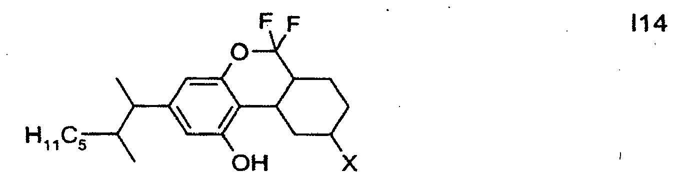 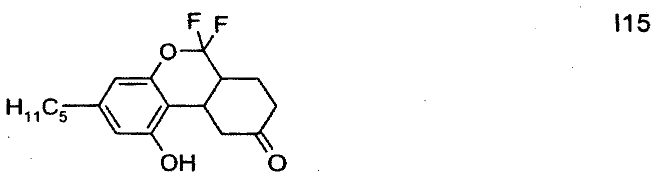 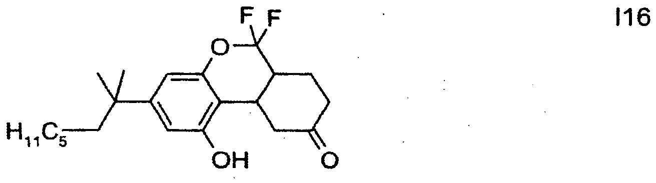 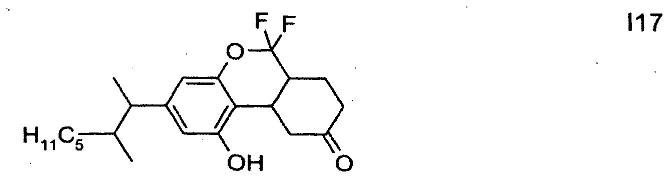 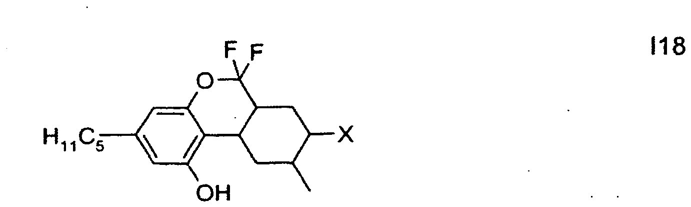 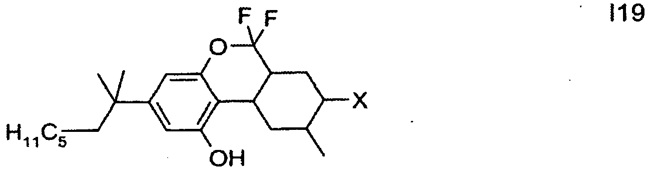 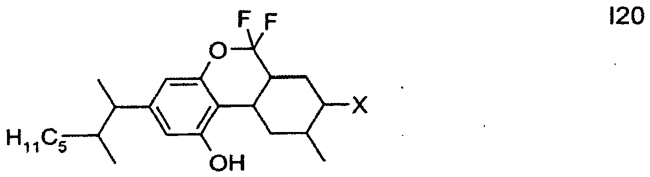 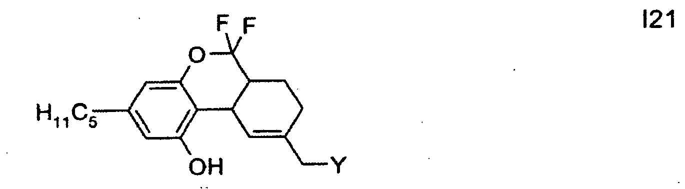 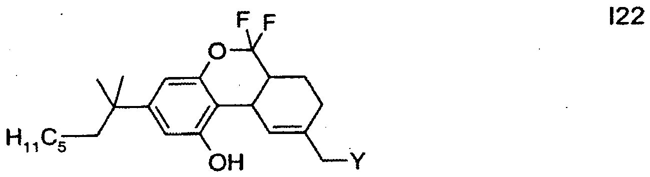 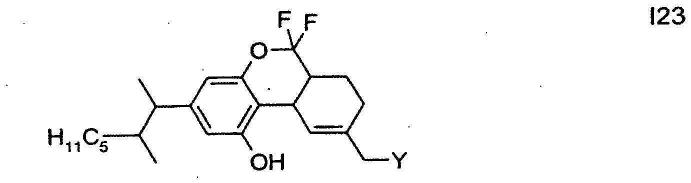 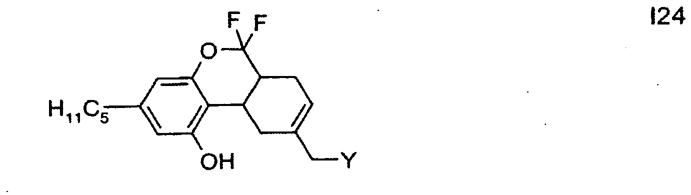 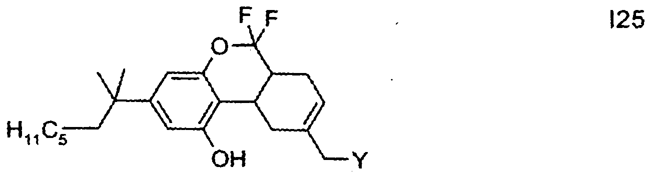 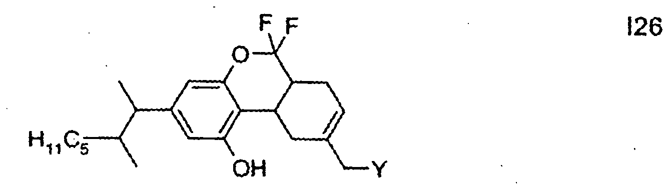 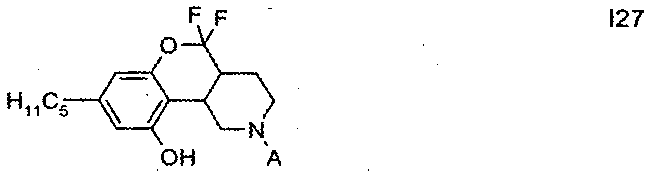 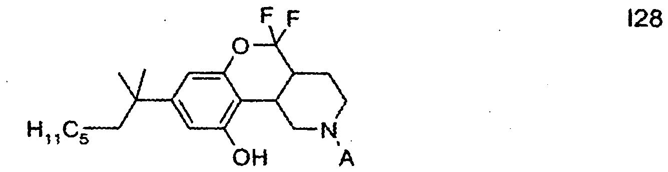 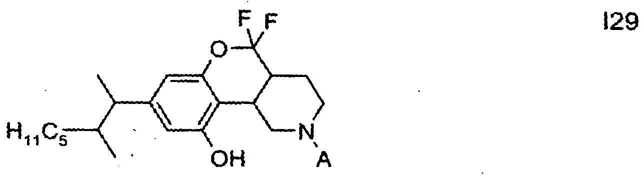 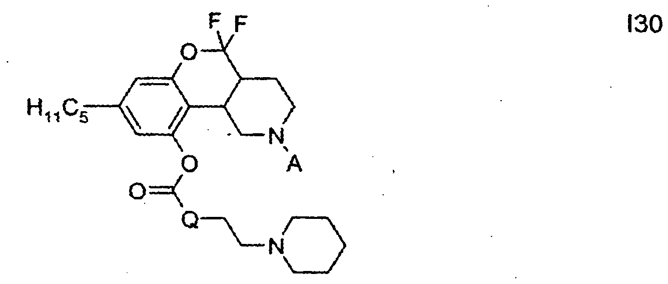 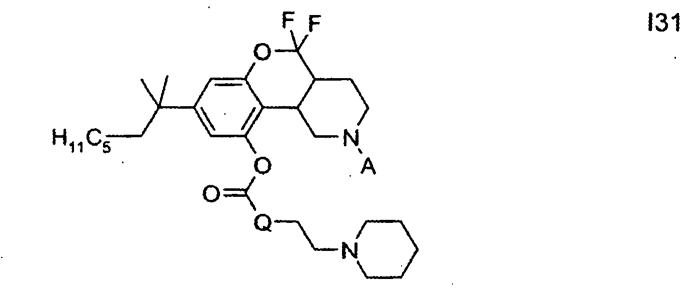 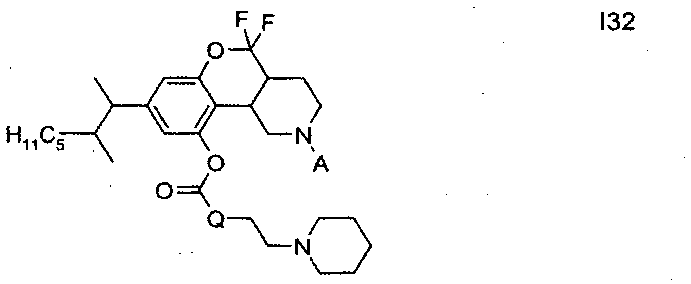 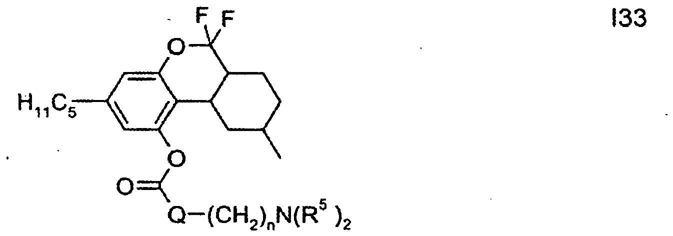 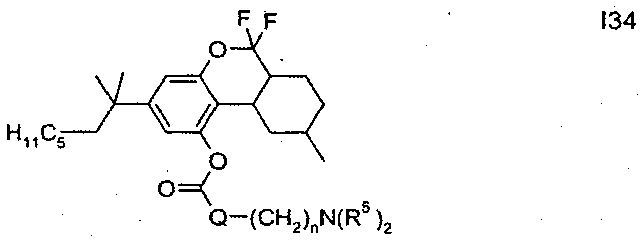 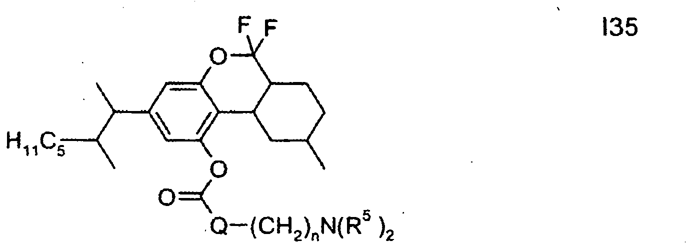 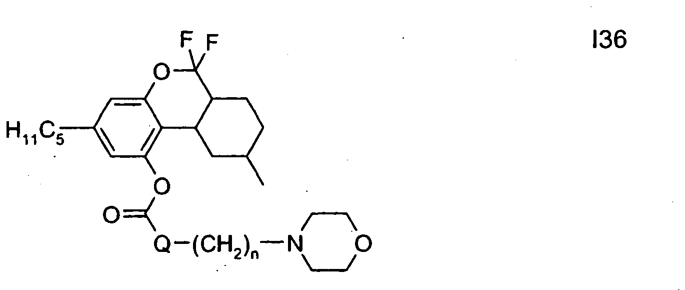 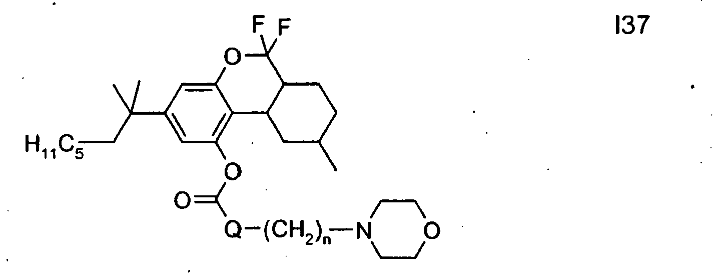 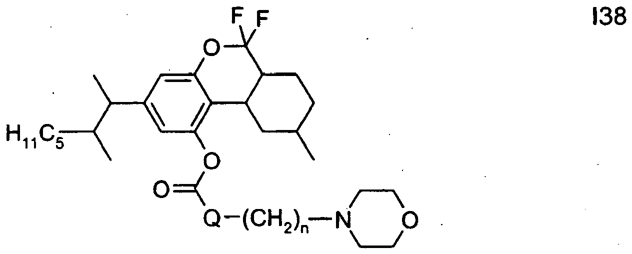 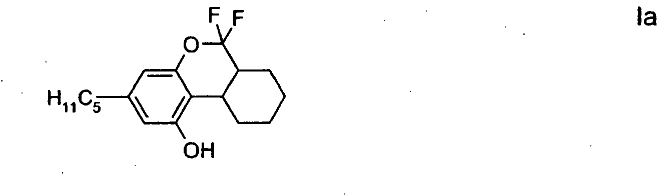 wobei
- X: OH, O-Alkyl, N(R⁵)₂
- Y: OH, O-Alkyl, COOR⁵ oder CON(R⁵)₂
und
- A: NHCH₂C≡C-R⁵ oder NHCH₂HC=CH-R⁵
bedeutet und R⁵ die oben angegebenen Bedeutungen aufweisen.

Die Erfindung bezieht sich zudem auf Verfahren zur Herstellung von Verbindungen der Formel I nach folgendem Schema: 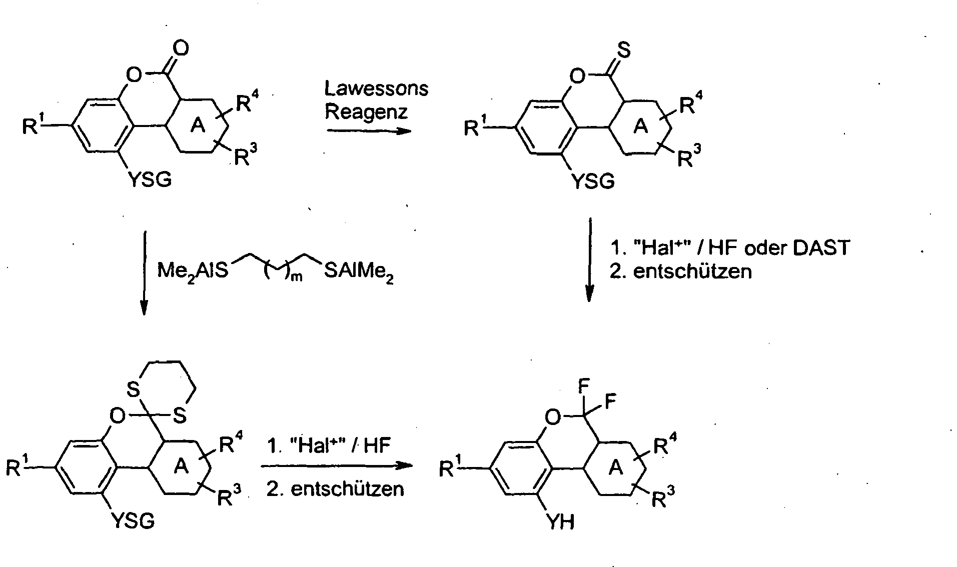 SG = Schutzgruppe
m = 0,1
Hal = Halogen

Die Ausgangsverbindungen für das erfindungsgemäße Verfahren sind die Verbindungen der Formel II 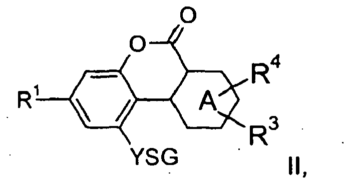 wobei R¹, R³, R⁴ und Y entsprechend der Endverbindungen der Formel I gewählt sind und SG eine Schutzgruppe, z. B. eine Ester- oder Amidbildende Gruppe wie Trialkylsilyl, Alkylcarbonyl-Gruppe oder Benzyl und insbesondere Trimethylsilyl, Acetyl, oder Benzyl bedeutet.

Auch die neuen Verbindungen der Formel II sind Gegenstand der vorliegenden Erfindung.

Die Erfindung betrifft somit ein Verfahren zur Herstellung von Verbindungen der Formel I durch Umsetzung von Verbindungen der Formel II, gekennzeichnet durch folgende Schritte:
a) Umsetzung der Verbindungen der Formel II mit aa) einem Thionierungsreagenz wie z. B. Lawesson Reagenz oder ab) mit einer Verbindung der Formel (Alkyl)₂Al-S-(CH₂)ₘ-S-Al(Alkyl)₂, worin Alkyl vorzugsweise Methyl, Ethyl, n-Propyl, iso-propyl, n-Butyl, sek-Butyl oder tert-Butyl und insbesondere Methyl und m 2 oder 3 bedeuten.
b) Oxidative Fluorierung der in aa) oder ab) erhaltenen Verbindungen mit einem Oxidationsmittel, das Haloniumequivalente freisetzt, wie z. B. 1,3-Dibrom-5,5-dimethylhydantoin (DBH) oder N-Iodsuccinimid in Gegenwart einer Fluoridquelle wie z. B. Triethylamin*3HF, oder durch Umsetzung der in ab) erhaltenen Thionolactone mit Fluorierungsreagenzien wie z. B. Diethylaminoschwefeltrifluorid oder Deoxofluor ^{®}, etc.; und nachfolgende Entschützung zu den Verbindungen der Formel I.

Desweiteren betrifft die Erfindung ein Verfahren zur Herstellung von Verbindungen der Formel I nach folgendem Schema: 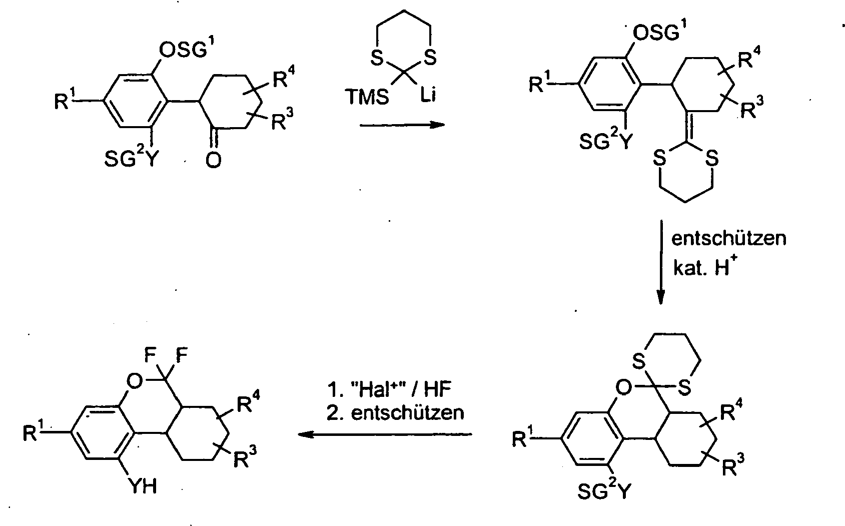 worin R¹, R³, und R⁴ entsprechend den Endverbindungen der Formel I gewählt ist.

Die Erfindung betrifft somit ein Verfahren zur Herstellung von Verbindungen der Formel I, gekennzeichnet durch folgende Schritte:
a) Umsetzung einer Verbindung der Formel III 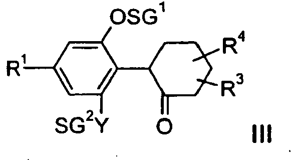 worin R¹, R³, und R⁴ die oben angegebene Bedeutung aufweisen, mit einer Verbindung der folgenden Formel

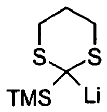

Oxidative Fluorierung der in a) erhaltenen Verbindungen mit einem Oxidationsmittel, das Haloniumequivalente freisetzt, wie z. B. 1,3-Dibrom-5,5-dimethylhydantoin (DBH) oder N-Iodsuccinimid in Gegenwart einer Fluoridquelle wie z. B. Triethylamin*3HF, und nachfolgende Entschützung zu den Verbindungen der Formel I.

Isomere können durch Chromatographie oder Kristallisation getrennt werden.

Die Umsetzungen erfolgen in der Regel in einem inerten Lösungsmittel.

Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.

Abgangsgruppen sind vorzugsweise Cl, Br, I oder eine freie oder eine reaktionsfähig abgewandelte OH-Gruppe wie z.B. ein aktivierter Ester, ein Imidazolid oder Alkylsulfonyloxy mit 1-6 C-Atomen (bevorzugt Methylsulfonyloxy oder Trifluormethylsulfonyloxy) oder Arylsulfonyloxy mit 6-10 C-Atomen (bevorzugt Phenyl- oder p-Tolylsulfonyloxy).
Derartige Reste zur Aktivierung der Carboxygruppe in typischen Acylierungsreaktionen sind in der Literatur (z.B. in den Standardwerken wie Houben-Weyl, Methoden der organischen Chemie, Georg-Thieme-Verlag, Stuttgart;) beschrieben.

Aktivierte Ester werden zweckmäßig in situ gebildet, z. B. durch Zusatz von HOBt oder N-Hydroxysuccinimid. Die Umsetzung erfolgt in der Regel in einem inerten Lösungsmittel, in Gegenwart eines säurebindenden Mittels vorzugsweise einer organischen Base wie DIPEA, Triethylamin, Dimethylanilin, Pyridin, N-Methylmorpholin, oder Chinolin oder eines Überschusses der umzusetzenden Carboxykomponente.

Auch der Zusatz eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums, Calciums oder Cäsiums kann günstig sein.

Die Reaktionszeit liegt je nach den angewendeten Bedingungen zwischen einigen Minuten und 14 Tagen, die Reaktionstemperatur zwischen etwa -30° und 140°, normalerweise zwischen -10° und 90°, insbesondere zwischen etwa 0° und etwa 70°.
Als inerte Lösungsmittel eignen sich die oben genannten.

Verbindungen der Formel I können ferner erhalten werden, indem man Verbindungen der Formel I aus einem ihrer funktionellen z. B. Schutzgruppen enthaltenden Derivate durch Behandeln mit einem solvolysierenden oder hydrogenolysierenden Mittel in Freiheit setzt.

Bevorzugte Ausgangsstoffe für die Solvolyse bzw. Hydrogenolyse sind solche, die sonst der Formel I entsprechen, aber anstelle einer oder mehrerer freier Amino- und/oder Hydroxygruppen entsprechende geschützte Amino- und/oder Hydroxygruppen enthalten, vorzugsweise solche, die anstelle eines H-Atoms, das mit einem N-Atom verbunden ist, eine Aminoschutzgruppe tragen, insbesondere solche, die anstelle einer HN-Gruppe eine R'-N-Gruppe tragen, worin R' eine Aminoschutzgruppe bedeutet, und/oder solche, die anstelle des H-Atoms einer Hydroxygruppe eine Hydroxyschutzgruppe tragen, z.B. solche, die der Formel I entsprechen, jedoch anstelle einer Gruppe -COOH eine Gruppe -COOR" tragen, worin R" eine Hydroxyschutzgruppe bedeutet.

Bevorzugte Ausgangsstoffe sind auch die Oxadiazolderivate, die in die entsprechenden Amidinoverbindungen überführt werden können.

Es können auch mehrere - gleiche oder verschiedene - geschützte Amino-und/oder Hydroxygruppen im Molekül des Ausgangsstoffes vorhanden sein. Falls die vorhandenen Schutzgruppen voneinander verschieden sind, können sie in vielen Fällen selektiv abgespalten werden.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte chemische Reaktion an anderen Stellen des Moleküls durchgeführt worden ist. Typisch für solche Gruppen sind insbesondere unsubstituierte oder substituierte Acyl-, Aryl-, Aralkoxymethyl- oder Aralkylgruppen. Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren in weitestem Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie POA; Alkoxycarbonyl wie Methoxycarbonyl, Ethoxycarbonyl, 2,2,2-Trichlorethoxycarbonyl, BOC (tert.-Butyloxycarbonyl), 2-lodethoxycarbonyl; Aralkyloxycarbonyl wie CBZ ("Carbobenzoxy"), 4-Methoxybenzyloxycarbonyl, FMOC; Arylsulfonyl wie Mtr. Bevorzugte Aminoschutzgruppen sind BOC und Mtr, ferner CBZ, Fmoc, Benzyl und Acetyl.

Der Ausdruck "Hydroxyschutzgruppe" ist ebenfalls allgemein bekannt und bezieht sich auf Gruppen, die geeignet sind, eine Hydroxygruppe vor chemischen Umsetzungen zu schützen, die aber leicht entfernbar sind, nachdem die gewünschte chemische Reaktion an anderen Stellen des Moleküls durchgeführt worden ist. Typisch für solche Gruppen sind die oben genannten unsubstituierten oder substituierten Aryl-, Aralkyl- oder Acylgruppen, ferner auch Alkylgruppen. Die Natur und Größe der Hydroxyschutzgruppen ist nicht kritisch, da sie nach der gewünschten chemischen Reaktion oder Reaktionsfolge wieder entfernt werden; bevorzugt sind Gruppen mit 1-20, insbesondere 1-10 C-Atomen. Beispiele für Hydroxyschutzgruppen sind u.a. Benzyl, 4-Methoxybenzyl, p-Nitrobenzoyl, p-Toluolsulfonyl, tert.-Butyl und Acetyl, wobei Benzyl und tert.-Butyl besonders bevorzugt sind.

Das In-Freiheit-Setzen der Verbindungen, der Formel I aus ihren funktionellen Derivaten gelingt - je nach der benutzten Schutzgruppe - z. B. mit starken Säuren, zweckmäßig mit TFA oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Die Anwesenheit eines zusätzlichen inerten Lösungsmittels ist möglich, aber nicht immer erforderlich. Als inerte Lösungsmittel eignen sich vorzugsweise organische, beispielsweise Carbonsäuren wie Essigsäure, Ether wie Tetrahydrofuran oder Dioxan, Amide wie DMF, halogenierte Kohlenwasserstoffe wie Dichlormethan, ferner auch Alkohole wie Methanol, Ethanol oder Isopropanol, sowie Wasser. Ferner kommen Gemische der vorgenannten Lösungsmittel in Frage. TFA wird vorzugsweise im Überschuß ohne Zusatz eines weiteren Lösungsmittels verwendet, Perchlorsäure in Form eines Gemisches aus Essigsäure und 70 %iger Perchlorsäure im Verhältnis 9:1. Die Reaktionstemperaturen für die Spaltung liegen zweckmäßig zwischen etwa 0 und etwa 50°, vorzugsweise arbeitet man zwischen 15 und 30° (Raumtemperatur).

Die Gruppen BOC, OBut und Mtr können z. B. bevorzugt mit TFA in Dichlormethan oder mit etwa 3 bis 5 N HCl in Dioxan bei 15-30° abgespalten werden, die FMOC-Gruppe mit einer etwa 5- bis 50 %igen Lösung von Dimethylamin, Diethylamin oder Piperidin in DMF bei 15-30°.

Hydrogenolytisch entfernbare Schutzgruppen (z. B. CBZ, Benzyl oder die Freisetzung der Amidinogruppe aus ihrem Oxadiazolderivat)) können z. B. durch Behandeln mit Wasserstoff in Gegenwart eines Katalysators (z. B. eines Edelmetallkatalysators wie Palladium, zweckmäßig auf einem Träger wie Kohle) abgespalten werden. Als Lösungsmittel eignen sich dabei die oben angegebenen, insbesondere z. B. Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt. Eine Hydrogenolyse der CBZ-Gruppe gelingt z. B. gut an 5 bis 10 %igem Pd/C in Methanol oder mit Ammomiumformiat (anstelle von Wasserstoff) an Pd/C in Methanol/DMF bei 20-30°.

Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Trifluormethylbenzol, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid, N-Methylpyrrolidon (NMP) oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.

Ester können z.B. mit Essigsäure oder mit NaOH oder KOH in Wasser, Wasser-THF oder Wasser-Dioxan bei Temperaturen zwischen 0 und 100° verseift werden.

Ferner kann man freie Aminogruppen in üblicher Weise mit einem Säurechlorid oder -anhydrid acylieren oder mit einem unsubstituierten oder substituierten Alkylhalogenid alkylieren, oder mit CH₃-C(=NH)-OEt umsetzen, zweckmäßig in einem inerten Lösungsmittel wie Dichlormethan oder THF und /oder in Gegenwart einer Base wie Triethylamin oder Pyridin bei Temperaturen zwischen -60 und +30°.

Eine Base der Formel I kann mit einer Säure in das zugehörige Säureadditionssalz übergeführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und -disulfonsäuren, Laurylschwefelsäure. Salze mit physiologisch nicht unbedenklichen Säuren, z.B. Pikrate, können zur Isolierung und /oder Aufreinigung der Verbindungen der Formel I verwendet werden.

Andererseits können Verbindungen der Formel I mit Basen (z.B. Natriumoder Kaliumhydroxid oder -carbonat) in die entsprechenden Metall-, insbesondere Alkalimetall- oder Erdalkalimetall-, oder in die entsprechenden Ammoniumsalze umgewandelt werden.
Auch physiologisch unbedenkliche organische Basen, wie z.B. Ethanolamin können verwendet werden.

Erfindungsgemäße Verbindungen der Formel I können aufgrund ihrer Molekülstruktur chiral sein und können dementsprechend in verschiedenen enantiomeren Formen auftreten. Sie können daher in racemischer oder in optisch aktiver Form vorliegen.

Da sich die pharmazeutische Wirksamkeit der Racemate bzw. der Stereoisomeren der erfindungsgemäßen Verbindungen unterscheiden kann, kann es wünschenswert sein, die Enantiomere zu verwenden. In diesen Fällen kann das Endprodukt oder aber bereits die Zwischenprodukte in enantiomere Verbindungen, durch dem Fachmann bekannte chemische oder physikalische Maßnahmen, aufgetrennt oder bereits als solche bei der Synthese eingesetzt werden.

Im Falle racemischer Amine werden aus dem Gemisch durch Umsetzung mit einem optisch aktiven Trennmittel Diastereomere gebildet. Als Trennmittel eignen sich z.B. optisch aktiven Säuren, wie die R- und S-Formen von Weinsäure, Diacetylweinsäure, Dibenzoylweinsäure, Mandelsäure, Äpfelsäure, Milchsäure, geeignet N-geschützte Aminosäuren (z.B. N-Benzoylprolin oder N-Benzolsulfonylprolin) oder die verschiedenen optisch aktiven Camphersulfonsäuren. Vorteilhaft ist auch eine chromatographische Enantiomerentrennung mit Hilfe eines optisch aktiven Trennmittels (z.B. Dinitrobenzoylphenylglycin, Cellulosetriacetat oder andere Derivate von Kohlenhydraten oder auf Kieselgel fixierte chiral derivatisierte Methacrylatpolymere). Als Laufmittel eignen sich hierfür wäßrige oder alkoholische Lösungsmittelgemische wie z.B. Hexan/Isopropanol/ Acetonitril z.B. im Verhältnis 82:15:3.

Die Erfindung umfasst nicht nur die genannten Verbindungen, sondern auch Mischungen und Zubereitungen, welche neben diesen erfindungsgemäßen Verbindungen auch andere pharmakologische Wirkstoffe oder Adjuvantien enthalten, die die primäre pharmakologische Wirkung der erfindungsgemäßen Verbindungen in gewünschter Weise beeinflussen können.

Die erfindungsgemäßen Verbindungen können als Arzneimittelwirkstoffe in der Human- oder Veterinärmedizin eingesetzt werden, insbesondere zur Prophylaxe oder Therapie von Krankheiten, die durch die zentralnervöse Wirkung der Verbindungen beeinflusst werden können.

Besonders bevorzugt können die erfindungsgemäßen Verbindungen zur Behandlung sexueller Störungen bzw. Steigerung der sexuellen Leistungsfähigkeit, von Durchfallerkrankungen, Nikotinabhängigkeit, entzündlicher ZNS-Erkrankungen (Demyelinisierung, virale Gehirnhautentzündung, Multiple Sklerose, Guillain-Barré-Syndrom) und unfallbedingter Gehimverletzungen oder Schädeltraumata, Appetenzstörungen, d. h. Abhängigkeiten verschiedener Art (Drogen, Alkohol, Zucker), Bulimie und ihre eventuellen Folgen (Fettleibigkeit, Diabetes) eingesetzt werden.

Sie sind weiterhin wirksam gegen Bluthochdruck oder wirken gegen Angstzustände und/oder Depressionen, als Sedativum, Tranquilizer, Analgeticum, Antiemeticum oder sie wirken entzündungshemmend.

Die zentralnervöse Wirkung kann durch die Verabreichung an Ratten in Dosierungen von 0.1-1000 mg/kg, bevorzugt von 1-100 mg/kg gezeigt werden. Es werden Effekte wie reduzierte spontane motorische Aktivität beobachtet, wobei die erforderliche Dosis sowohl von der Wirksamkeit der Verbindung als auch von Körpergewicht des Versuchtieres abhängt.

Gegenstand der Erfindung sind dementsprechend Verbindungen der oben und unten sowie in den Ansprüchen definierten Formeln einschließlich ihrer physiologisch unbedenklichen Salze als Arzneimittel, Diagnostika oder Reagenzien.

Gegenstand sind auch entsprechende pharmazeutische Zubereitungen, welche mindestens ein Arzneimittel der Formel I sowie gegebenenfalls Träger- und/oder Hilfsstoffe enthalten. Als Trägerstoffe kommen organische oder anorganische Substanzen in Frage, die sich für die enterale (z. B. orale), parenterale, topische Applikation oder für eine Applikation in Form eines Inhalations-Sprays eignen und mit den neuen Verbindungen nicht reagieren, beispielsweise Wasser, pflanzliche Öle, Benzylalkohole, Alkylenglykole, Polyethylenglykole, Glyzerintriacetat, Gelatine, Kohlenhydrate wie Lactose oder Stärke, Magnesiumstearat, Talk, Vaseline. Zur oralen Anwendung dienen insbesondere Tabletten, Pillen, Dragees, Kapseln, Pulver, Granulate, Sirupe, Säfte oder Tropfen, zur rektalen Anwendung Suppositorien, zur parenteralen Anwendung Lösungen, vorzugsweise ölige oder wässrige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z. B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und/oder mehrere weitere Wirkstoffe enthalten, z. B. ein oder mehrere Vitamine.

Für die Applikation als Inhalations-Spray können Sprays verwendet werden, die den Wirkstoff entweder gelöst oder suspendiert in einem Treibgas oder Treibgasgemisch (z. B. CO₂) enthalten. Zweckmäßig verwendet man den Wirkstoff dabei in mikronisierter Form, wobei ein oder mehrere zusätzliche physiologisch verträgliche Lösungsmittel zugegen sein können, z. B. Ethanol. Inhalationslösungen können mit Hilfe üblicher Inhalatoren verabreicht werden.

Die erfindungsgemäßen Substanzen können in der Regel in Analogie zu anderen, im Handel befindlichen THC-Analoga verabreicht werden, vorzugsweise in Dosierungen zwischen etwa 0,05 und 500 mg, insbesondere zwischen 0,5 und 100 mg pro Dosierungseinheit. Die tägliche Dosierung liegt vorzugsweise zwischen etwa 0,01 und 20 mg/kg Körpergewicht. Die spezielle Dosis für jeden Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabreichungszeitpunkt und -weg, von der Ausscheidungsgeschwindigkeit, Arzneistoffkombination und Schwere der jeweiligen Erkrankung, welcher die Therapie gilt.

Ferner können die neuen Verbindungen der Formel I in der analytischen Biologie und Molekularbiologie verwendet werden.

Die folgenden Beispiele sollen die Erfindung erläutern .

### Beispiele

### 1. (6aR*,10aS*)-6,6-Difluor-3-methyl-6a,7,8,9,10,10a-hexahydro-6H-benzo[c]chromen-1-ol

### 1.1 1-Hydroxy-3-pentyl-7,8,9,10-tetrahydrobenzo[c]chromen-6-on

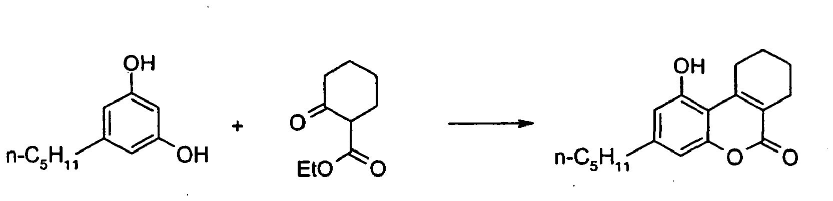

Olivetol (49,5 g, 261 mmol) und 2-Oxo-cyclohexancarbonsäureethylester (47,7ml, 285 mmol) wurden in Toluol vorgelegt und nach Zugabe von 28,8 ml (310 mmol) Phosphorylchlorid 38 h bei Raumtemp. gerührt. Das Reaktionsgemisch wurde langsam auf 400 ml Wasser gegossen und 30 min kräftig gerührt. Der ausgefallene gelbe Feststoff wurde abfiltriert und mit Wasser und Toluol gewaschen. Der hellgelbe Feststoff wurde in heißem Ethylmethylketon aufgenommen, mit wäßriger gesättigter Natriumhydrogencarbonatlsg. Lösung und ges. Kochsalzlösung gewaschen, über Natriumsulfat getrocknet, filtriert und i. Vak. eingeengt. Kristallisation des Rohproduktes aus Acetonitril/Toluol lieferte 32,7 g (82 %) 1-Hydroxy-3-pentyl-7,8,9,10-tetrahydrobenzo[c]chromen-6-on als farblose Kristalle.

### 1.2 1-tert-Butyldimethylsilyloxy-3-pentyl-7,8,9,10-tetrahydro-benzo[c]-chromen-6-on

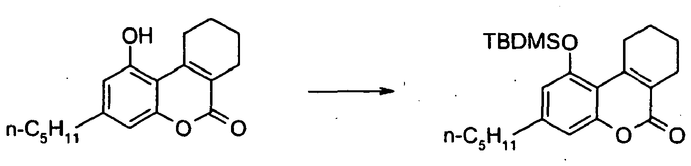

32 g (112 mmol) 1-Hydroxy-3-pentyl-7,8,9,10-tetrahydrobenzo[c]chromen-6-on wurden in 200 ml DMF vorgelegt, 10 g Imidazol (145 mmol) zugegeben, bei 0°C eine Lösung von 21 g (135 mmol) tert-Butyldimethylsilylchlorid in 100 ml DMF zugetropft und 20 h bei Raumtemp. gerührt. Anschließend wurde der Ansatz mit MTB-Ether versetzt, mit Wasser gewaschen und über Natriumsulfat getrocknet. Das Lösungsmittel wurde i. Vak. entfernt und der Rückstand durch Chromatographie mit Heptan/Essigester (6:1) aufgereinigt. Man erhielt 44,5 g (94 %) 1-tert-Butyldimethylsilyloxy-3-pentyl-7,8,9,10-tetrahydrobenzo[c]chromen-6-on als gelben Feststoff.

### 1.3 (6aR*,10aS*)-1-tert-Butyldimethylsilyloxy-3-pentyl-6a,7,8,9,10,10a-hexahydro-benzo[c]chromen-6-on

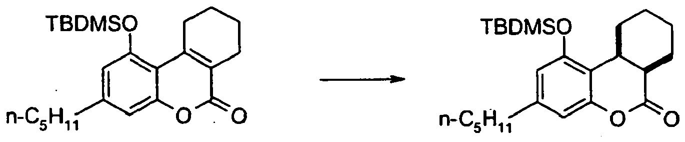

30g (74,9mmol) 1-tert-Butyldimethylsilyloxy-3-pentyl-7,8,9,10-tetrahydrobenzo[c]-chromen-6-on wurden in THF an Palladiumkohle bis zum Stillstand hydriert. Der Katalysator wurde abfiltriert, das Lösungsmittel i. Vak. entfernt und der Rückstand mit Heptan/Essigester (15:1) über Kieselgel filtriert. Man erhielt 24.5 g (81 %) (6aR*,10aS*)-1-tert-Butyldimethylsilyloxy-3-pentyl-6a,7,8,9,10,10a-hexahydro-benzo[c]-chromen-6-on als farbloses Öl.

### ¹H-NMR (300 MHz, CDCl₃)

δ = 6,47 ppm (d, ⁴J=1,3 Hz, 1 H), 6,39 (d, ⁴J=1,3 Hz, 1 H), 3,11 (ddd, J_{cis}=3,7 Hz, J_{cis}=5,8 Hz, Jₜᵣₐₙₛ=12,3 Hz, 1H), 2,87-2,82 (m, 1H), 2,50 (t, ³J=7,0Hz, 2H), 1,82 (bt, ³J=6,0 Hz, 2H), 1,66-1,49 (m, 6H), 1,36-1,15 (m, 6H), 1,02 (s, 9H), 0,89 (t, ³J=7,0 Hz, 3H), 0,26(s, 3H), 0,25 (s, 3H)

### 1.4. (6aR*,10aS*)-6,6-Difluor-3-methyl-6a,7,8,9,10,10a-hexahydro-6H-benzo[c]chromen-1-ol

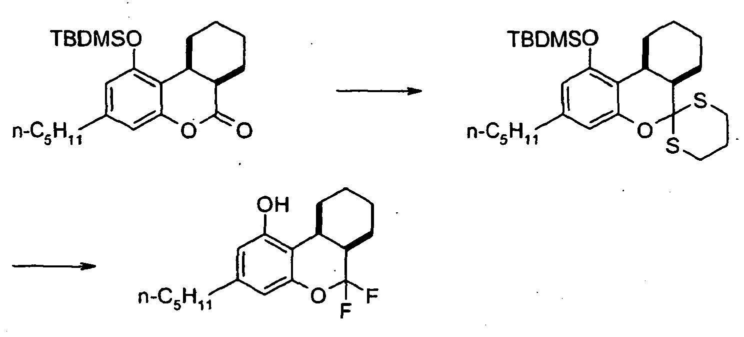

75 ml Dichlormethan wurden auf -78°C abgekühlt, Trimethylaluminium (2M. Lösung in Hexan, 16,25 ml, 32,5 mmol) zugegeben und 1,57 ml (15,7mmol) 1,3-Propandithiol langsam zugetropft. Der Ansatz wurde weitere 5 min gerührt und dann innerhalb von 30 min auf Raumtemp. erwärmt, 30 min rühren gelassen und erneut auf -78°C gekühlt. Anschließend wurde eine Lösung von 5,6 g (13 mmol) (6aR*,10aS*)-1-tert-Butyldimethylsilyloxy-3-pentyl-6a,7,8,9,10,10a-hexahydrobenzo[c]-chromen-6-on in 25 ml Dichlormethan langsam zugetropft, der Ansatz auftauen gelassen und 18 h bei Raumtemp. nachgerührt. Nach Hydrolyse wurde die wäßrige Phase mit Dichlormethan extrahiert, die vereinigten organischen Extrakte über Natriumsulfat getrocknet und abfiltriert. Die Lösung wurde 2 h in Gegenwart von 30 g Kieselgel gerührt, filtriert und eingeengt.
17,7 g (62 mmol) DBH wurde in 150 ml Dichlormethan vorgelegt, bei -78°C 1,9 ml (62mmol) 60proz. HF-Pyridin zugegeben und die Suspension 30 min bei dieser Temperatur gerührt. Anschließend wurde eine Lösung der im ersten Schritt erhaltenen Dithiospiroverbindung in 50 ml Dichlormethan langsam zugetropft und 3,5 h bei -70°C gerührt. Nach Hydrolyse mit 10proz. Natronlauge wurde der Ansatz mit Dichlormethan extrahiert, mit ges. Natriumchloridlsg. gewaschen und über Natriumsulfat getrocknet.

Das Lösungsmittel wurde i. Vak. entfernt und der Rückstand mit Heptan/Essigester (20:1) an Kieselgel chromatographiert. Das so erhaltene Rohprodukt wurde in Tetrahydrofuran/Wasser 2:1 in Gegenwart von Triethylamin an Palladium-Aktivkohle bis zum Stillstand hydriert, filtriert und mit Wasser gewaschen. Die wäßrigen Phasen wurden mit Dichlormethan extrahiert und die vereinigten org. Phasen wurden über Natriumsulfat getrocknet. Das Lösungsmittel wurde i. Vak. entfernt und der Rückstand mit Heptan/Essigester (15:1) an Kieselgel chromatographiert. Man erhielt (6aR*,10aS*)-6,6-Difluor-3-pentyl-6a,7,8,9,10,10a-hexahydro-6H-benzo[c]-chromen-1-ol als farbloses Öl

### ¹H-NMR (300 MHz, CDCl₃)

δ = 6,39 (d, ⁴J=1,5 Hz, 1 H), 6,22 (d, ⁴J=1,5 Hz, 1 H), 4,73 (s, 1 H), 3,28-3,18 (m, 1 H), 2,74 (bt, J=1,9 Hz, 1 H), 2,46 (t, ³J=7,0 Hz, 2H), 2,17-2,10 (m, 1 H), 1,93-1,83 (m, 3H), 1,61-1,25 (m, 10H), 0,89 (t, ³J=7,0 Hz, 3H)

### ¹⁹F-NMR (235 MHz CDCl₃):

δ = -79,70 (dd, ²J_{F,F}=151,4Hz, ³J_{H,F}=21,8Hz, 1 F), -81,06 (d, ²J_{F,F}=151,4Hz, 1 F)

### 2. (6aR*,10aS*)-3-(1,1-Dimethylheptyl)-6,6-difluor-6a,7,8,9,10,10a-hexahydro-6H-benzo[c]chromen-1-ol

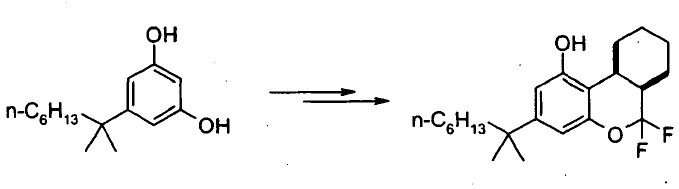

In Analogie zu der unter 1.1 bis 1.3 beschriebenen Synthese erhielt man aus 5-(1,1-Dimethylheptyl)resorcin das (6aR*,10aS*)-3-(1,1-Dimethyl-heptyl)-6,6-difluor-6a,7,8,9,10,10a-hexahydro-6H-benzo[c]chromen-1-ol.

### ¹H-NMR (300 MHz, CDCl₃):

δ = 6,51 ppm (d, ⁴J=1,8 Hz, 1 H), 6,34 (d, ⁴J=1,8 Hz, 1 H), 4,83 (s, 1 H), 3,28-3,19 (m, 1H), 2,74 (bt, J=11,3 Hz, 1H), 2,17-2,10 (m, 1H), 1,94-1,81 (m, 4H), 1,55-1,36 (m, 8H), 1,25-1,15 (m, 8H), 1.12-1,01 (m, 3H), 0,85 (t, ³J=6,8 Hz, 3H)

### ¹⁹F-NMR (235 MHz, CDCl₃):

δ = -79,7 (dd, ²J_{F,F}=152 Hz, ³J_{H,F}=22,4 Hz, 1 F), -81,1 (d, ²J_{F,F}=152 Hz, 1 F)

### 3. (6aR*,10aR*)-6,6-Difluor-3-pentyl-6a,7,8,9,10,10a-hexahydro-6H-benzo[c]chromen-1-ol

*3.1 2-(2,6-Dimethoxy-4-pentyl-phenyl)-cyclohexancarbonsäuremethylester* 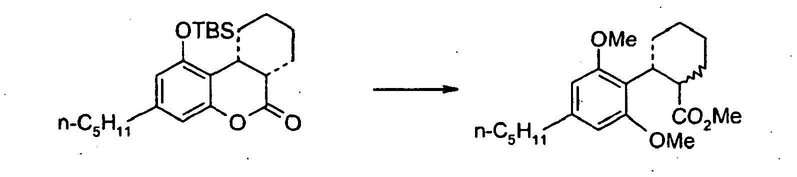 28,5g (93,9 mmol) 1-tert-Butyldimethylsilyloxy-3-pentyl-6a,7,8,9,10,10a-hexahydro-benzo[c]-chromen-6-on wurden in 600 ml Methanol gelöst und nach Zugabe von 15,9 g (283 mmol) Kaliumhydroxid 3 h unter Rückfluß erhitzt. Das Lösungsmittel wurde i. Vak. entfernt, der Rückstand in 450 ml THF und 100 ml DMPU aufgenommen und in Gegenwart von 5 Äquivalenten Methyliodid über Nacht unter Rückfluß erhitzt. Nach Zugabe von 150 ml Wasser wurde die org. Phase abgetrennt und die wäßrige Phase dreimal mit MTB-Ether extrahiert. Die vereinigten org. Phasen wurden mit Wasser gewaschen und über Natriumsulfat getrocknet. Das Lösungsmittel wurde i. Vak. entfernt und der Rückstand mit Heptan/Essigester (8:1) über Kieselgel filtriert. Man erhielt 27,5 g (84%) der diastereomeren 2-(2,6-Dimethoxy-4-pentyl-phenyl)-cyclohexancarbonsäuremethylester als gelbes Öl.

### 3.2 trans-2-(2,6-Dimethoxy-4-pentylphenyl)cyclohexancarbonsäuremethylester

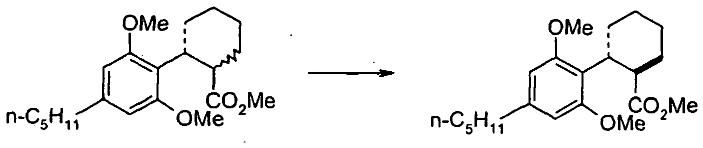

600 mg (1,7 mmol) 2-(2,6-Dimethoxy-4-pentyl-phenyl)-cyclohexancarbonsäuremethylester wurden in 10 ml Methanol gelöst, eine Lösung von 200 mg (8,7 mmol) Natrium in 7,5 ml Methanol hinzugefügt und über Nacht unter Rückfluß erhitzt. Anschließend wurde mit Wasser verdünnt, mit Essigsäure angesäuert und mit Dichlormethan extrahiert. Die vereinigten organischen Phasen wurden über Natriumsulfat getrocknet, filtriert und eingeengt. Man erhielt 388 mg (75 %) trans-2-(2,6-Dimethoxy-4-pentyl-phenyl)-cyclohexancarbonsäuremethylester als farbloses Öl.

### 3.3 (6aR*, 10aR*)-1-Hydroxy-3-pentyl-6a,7,8,9,10,10a-hexahydrobenzo[c]chromen-6-on

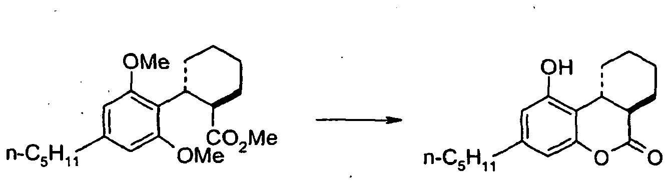

260 mg (0,75 mmol) trans-2-(2,6-Dimethoxy-4-pentyl-phenyl)-cyclohexancarbon-säuremethylester wurden in 10 ml Dichlormethan vorgelegt und bei -70°C mit 191 µl (2mmol) Bortribromid versetzt. Nach 20 min wurde die Kühlung entfernt und der Ansatz bei 0 °C mit ges. Natriumhydrogencarbonatlsg. hydrolysiert. Die Mischung wurde mit Dichlormethan extrahiert, über Natriumsulfat getrocknet und eingeengt. Chromatographie mit Heptan/Essigester (7:1) lieferte 100 mg (45 %) (6aR*,10aR*)-1-Hydroxy-3-pentyl-6a,7,8,9,10,10a-hexahydro-benzo[c]-chromen-6-on als farblosen Feststoff.

### ¹H-NMR (300 MHz, CDCl₃):

δ = 6,48 (d, ⁴J=1,6 Hz, 1 H), 6,31 (d, ⁴J=1,6 Hz, 1H), 4,94 (s, 1H), 3,28-3,19 (m, 1H), 2,82 (dt, Jₜᵣₐₙₛ=12,4 Hz, J_{cis}=3,6Hz 1 H), 2,49 (t, ³J=7,0 Hz, 2H), 2,38-2,18 (m, 2H), 1,92-1,85 (m, 2H), 1,60-1,47 (m, 3H), 1,37-1,25 (m, 7H), 0,89 (t, ³J=7,0Hz, 3H).

### 3.4. (6aR*10aR*)-1-(tert-Butyldimethylsilyloxy)-3-pentyl-6a,7,8,9,10,10a-hexahydro-benzo[c]chromen-6-on

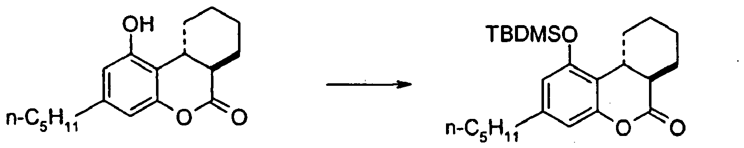

Umsetzung von (6aR*,10aR*)-1-Hydroxy-3-pentyl-6a,7,8,9,10,10a-hexahydrobenzo[c]chromen-6-on entsprechend der unter 1.2 beschriebenen Synthese lieferte (6aR*,10aR*)-1-(tert-Butyldimethylsilyloxy)-3-pentyl-6a,7,8,9,10,10a-hexahydrobenzo[c]chromen-6-on als farbloses Öl.

### 3.5 (6aR*,10aR*)-1-(tert-Butyldimethylsilyloxy)-3-pentyl-6a,7,8,9,10,10a-hexahydro-benzo[c]chromen-6-thion

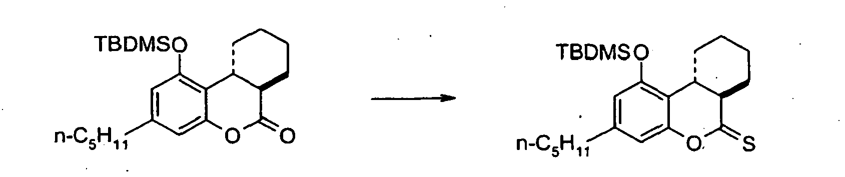

2. 00 g (4,97 mmol) (6aR*,10aR*)-1-(tert-Butyldimethylsilyloxy)-3-pentyl-6a,7,8,9,10,10a-hexahydro-benzo[c]chromen-6-on wurde mit 4,25 g (10.2 mmol) Lawessons Reagenz in Xylol 17 h bei 140°C unter Rückfluß erhitzt. Das Lösungsmittel wurde i. Vak. entfernt und der Rückstand mit Heptan/Essigester (20:1) an Kieselgel chromatographiert. Man erhielt (6aR*,10aR*)-1-(tert-Butyldimethylsilyytoxy)-3-pentyl-6a,7,8,9,10,10a-hexahydro-benzo[c]chromen-6-thion als gelbes Öl.

### ¹H-NMR (300 MHz, CDCl₃):

δ = 6,60 (d, ⁴J=1,3Hz, 1 H), 6,43 (d, ⁴J=1,3Hz, 1H), 3,08 (ddd, J_{cis}=3,5Hz, J_{cis}=5,8Hz, Jₜᵣₐₙₛ=12,2Hz, 1H), 2,86-2,80 (m, 2H), 2,52 (t, ³J=7,0Hz, 2H), 1,87-1,70 (m, 4H), 1,61-1,53 (m, 3H), 1,36-1,25 (m, 6H), 1,02 (s, 9H), 0,89 (t, ³J=7,0Hz, 3H), 0,26(s, 3H), 0,25 (s, 3H).

### 3.6 (6aR*,10aR*)-6,6-Difluor-3-pentyl-6a,7,8,9,10,10a-hexahydro-6H-benzo[c]chromen-1-ol

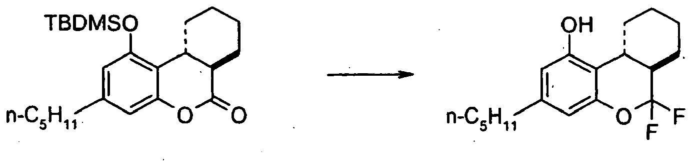

500 mg (1,19 mmol) (6aR*,10aR*)-1-(tert-Butyldimethylsilyloxy)-3-pentyl-6a,7,8,9,10,10a-hexahydro-benzo[c]chromen-6-thion wurden in 10 ml Dichlormethan gelöst und unter Eiskühlung mit 0,4 ml (3 mmol) DAST versetzt. Die Kühlung wurde entfernt und der Ansatz über Nacht bei Raumtemp. rühren gelassen. Anschließend wurde der Ansatz auf ges. Natriumhydrogencarbonatlsg. gegeben und dreimal mit MTB-Ether extrahiert. Die vereinigten org. Phasen wurden mit Wasser gewaschen und über Natriumsulfat getrocknet. Das Lösungsmittel wurde i. Vak. entfernt und der Rückstand mit Heptan/Essigester (15: 1) an Kieselgel chromatographiert. Man erhielt (6aR*,10aR*)-6,6-Difluor-3-pentyl-6a,7,8,9,10,10a-hexahydro-6H-benzo[c]chromen-1-ol als farbloses Öl

### 4. (6aR*,10aS*)-6,6-Difluor-1-hydroxy-3-pentyl-6,6a,7,8,10,10a-hexahydrobenzo-[c]chromen-9-on

### 4.1 -1-(tert-Butyldimethylsilyl)-3-pentyl-7,10-dihydro-8H-benzo[c]chromen-6,9-dion-9-ethylenketal

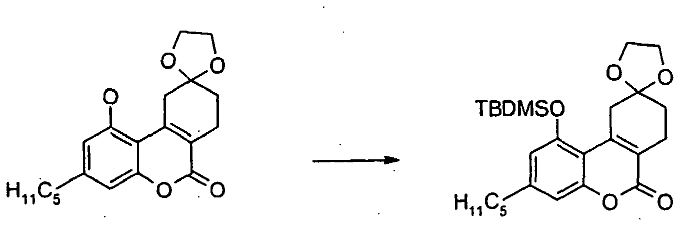

In Analogie zu der unter 1.2 beschriebenen Synthese erhielt man aus -1-Hydroxy-3-pentyl-7,10-dihydro-8H-benzo[c]chromen-6,9-dion-9-ethylenketal [hergestellt nach R.A. Archer, W.B. Blanchard, W.A. Day, D.W. Johnson, E.R. Lavagnino, C.W. Ryan, J. Org. Chem. 1977, 42, 2277-2284] und tert-Butyldimethylsilylchlorid das 1-(tert-Butyldimethylsilyloxy)-3-pentyl-7,10-dihydro-8H-benzo[c]chromen-6,9-dion-9-ethylenketal.

### 4.2 (6aR*,10aS*)-1-(tert-Butyldimethylsilyloxy)-3-Pentyl-7,8,10,10a-tetrahydro-6aH-benzo[c]chromen-6,9-dion-ethylenketal

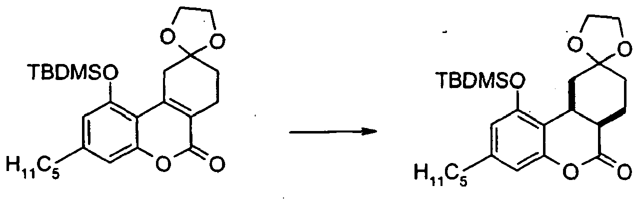

In Analogie zu der unter 1.3 beschriebenen Synthese lieferte Hydrierung des 1-(tert-Butyldimethylsilyloxy)-3-pentyl-7,10-dihydro-8H-benzo[c]chromen-6,9-dion-9-ethylenketals an Palladium-Aktivkohle das (6aR*,10aS*)-1-(tert-Butyldimethylsilyloxy)-3-pentyl-7,8,10,10a-tetrahydro-6aH-benzo[c]chromen-6,9-dion-9-ethylenketal.

### 4.3 (6aR*,10aS*)-1-(tert-Butyldimethylsilyloxy)-3-pentyl-7,8,10,10a-tetrahydro-6aH-benzo[c]chromen-6,9-dion-6-propylendithioketal-9-ethylenketal .

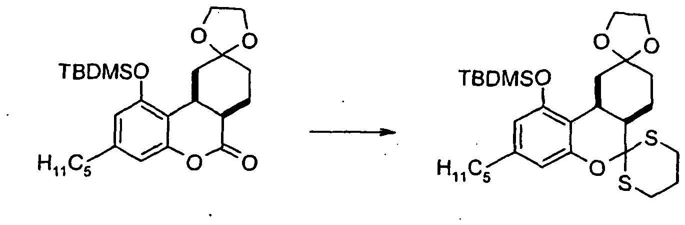

150 ml Dichlormethan wurden auf -78°C abgekühlt, 26 ml (51 mmol) einer 2 M Lösung von Trimethylaluminium in Hexan zugegeben und 2,5 ml (25 mmol) 1,3-Propandithiol langsam zugetropft. Der Ansatz wurde weitere 5 min gerührt und dann innerhalb von 30 min auf Raumtemp. erwärmt, 30 min rühren gelassen und erneut auf -78°C gekühlt. Anschließend wurde eine Lösung von 9,9 g (21 mmol) (6aR*,10aS*)-1-(tert-Butyldimethylsilyloxy)-3-pentyl-7,8,1 0,10a-tetrahydro-6aH-benzo[c]chromen-6,9-dion-9-ethylenketal in 50 ml Dichlormethan langsam zugetropft, der Ansatz auftauen gelassen und 18 h bei Raumtemp. nachgerührt. Nach Hydrolyse wurde die wäßrige Phase mit Dichlormethan extrahiert und die vereinigten organischen Phasen über Natriumsulfat getrocknet. Das Lösungsmittel wurde i. Vak. entfernt, der Rückstand in Dichlormethan gelöst und 2 min Gegenwart von 350 mg Pyridinium-p-toluolsulfonat gerührt. Die Lösung wurde mit Wasser gewaschen und die wäßrige Phase mit Dichlormethan extrahiert. Die vereinigten organischen Phasen wurden mit ges. Natriumhydrogencarbonatlsg. gewaschen, über Natriumsulfat getrocknet, filtriert und eingeengt. Chromatographie an Kieselgel mit Heptan/Essigester (8:1, 3:1) lieferte (6aR*,10aS*)-1-(tert-Butyldimethylsilyloxy)-3-pentyl-7,8,10,10a-tetrahydro-6aH-benzo[c]chromen-6,9-dion-6-propylendithioketal-9-ethylenketal als farbloses Öl.

### ¹H-NMR (300 MHz, CDCl₃):

δ = 6,32 (d, ⁴J=1,1 Hz, 1H), 6,21 (d, ⁴J=1,1 Hz, 1H), 3,82-3,73 (m, 4H), 3,01-2,97 (m, 1 H), 2,93-2,85 (m, 3H), 2,44 (t, ³J=7,0 Hz, 2H), 2,25-2,20 (m, 2H), 2,15-2,11 (m, 2H), 1,99 (dt, J=14,0 Hz, J=5,2 Hz, 1H), 1,86 (dd, J=12,2 Hz, J=3,0 Hz, 1H), 1,72 (dt, J=14,0 Hz, J=5,2 Hz, 1H), 1,58-1,52 (m, 5H), 1,34-1,25 (m, 4H), 0,96 (s, 9H), 0,88 (t, ³J=7,0 Hz, 3H), 0,35(s, 3H), 0,27 (s, 3H).

### 4. 4 (6aR*10aS*)-6,6-Difluor-1-hydroxy-3 pentyl-6,6a,7,8,10,10a-hexahydrobenzo[c]-chromen-9-on

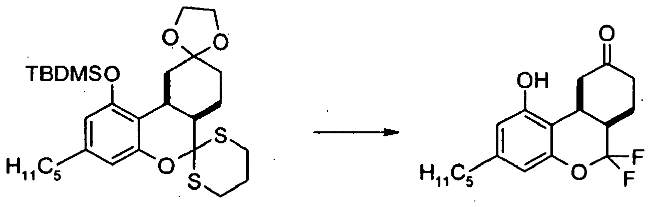

1.82 g (3,30 mmol) der in 4.3 erhaltenen Thioverbindung wurden bei -78 °C zu einer Suspension von 3.35 g (14.6 mmol) N-Iodsuccinimid in 100 ml Dichlormethan und 0.6 ml (21 mmol) 70proz. HF-Pyridinkomplex gegeben, 3 h rühren gelassen und anschließend auf eine Mischung aus 100 ml 10proz. Natronlauge und 20 ml ges. Natriumhydrogensulfitlsg gegeben. Die wäßrige Phase wurde abgetrennt und mit Dichlormethan extrahiert. Die vereinigten org. Phasen wurden mit ges. Natriumchloridlsg. gewaschen und über Natriumsulfat getrocknet. Das Lösungsmittel wurde i. Vak. entfernt, der Rückstand in Aceton gelöst und in Gegenwart von p-Toluolsulfonsäure 24 h bei Raumtemp. rühren gelassen. Die Lösung wurde mit Natronlauge neutralisiert und das Lösungsmittel i. Vak. entfernt. Chromatographie des Rohproduktes mit Heptan/Essigester (3:1) an Kieselgel lieferte das (6aR*,10aS*)-6,6-Difluor-1-hydroxy-3-pentyl-6,6a,7,8,10,10a-hexahydrobenzo[c]chromen-9-on als gelbes Öl.

### ¹⁹F-NMR (235 MHz, CDCl₃):

δ = -81,0 ppm (²J = 152 Hz, 1 F), -82,5 (²J = 152 Hz, ³J = 20 Hz, 1F).

Die spezifische Ligandenbindung an die Rezeptoren wird definiert als die Differenz zwischen vollständiger Bindung und nicht spezifischer Bindung, die in Gegenwart von einem Überschuss ungelabellten Liganden ermittelt wird (siehe z. B. MUNRO, S., THOMAS, K.L. and ABU-SHAAR, M. (1993), Molecular characterization of a peripheral receptor for cannabinoids. Nature, 365: 61-65*.* RINALDI-CARMONA, M., CALANDRA, B., SHIRE, D., BOUABOULA, M., OUSTRIC, D., BARTH, F., CASELLAS, P., FERRARA, P. and LE FUR, G. (1996), Characterization of two cloned human CB1 cannabinoid receptors isoform; J. Pharmacol. Exp. Ther., 278: 871-878*.*

Diese Ergebnisse werden ausgedruckt als Prozentanteil der spezifischen Vergleichsbindung in Gegenwart der Testsubstanz Ia.

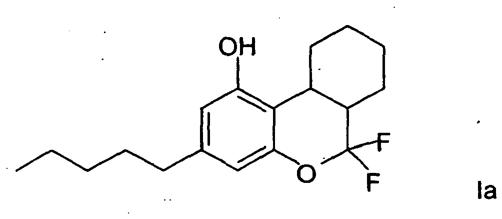

Die IC₅₀ Werte (Konzentration, die für die halb-maximale Inhibierung der spezifischen Kontrollbindung erforderlich ist) und Hill-Koeffizienten (*n_{H}*) werden durch nicht lineare Regressionsanalysen der Competitionskurven erhalten wobei die Kurvenanpassung nach der Hill-Gleichung erfolgt.

Die Inhibitionskonstanzen (Kᵢ) werden nach der Cheng Prusoff Gleichung errechnet (Kᵢ = IC₅₀/1+(L/K_{D})).

Die Werte IC₅₀ und Kᵢ bestimmt für die Verbindung Ia sind in der folgenden Tabelle 1 angegeben.

**Tabelle 1**

| Assay | Ligand | IC₅₀ (M) | Kᵢ (M) | *n_{H}* |
|---|---|---|---|---|
| CB1 (*h*) | | | | |
| | Verbindung Ia | 2.2E-06 | 1.9E-06 | 1.5 |
| CB2 (*h*) | | | | |
| | Verbindung Ia | 1.4E-06 | 9.2E-07 | 0.9 |

**Tabelle 2 Die Daten der Vergleichssubstanzen sind in der folgenden Tabelle angegeben.**

| Assay | Ligand | IC₅₀ (M) | Kᵢ (M) | *n_{H}* |
|---|---|---|---|---|
| CB1 (*h*) | | | | |
| | CP55940 | 2.2E-09 | 1.9E-09 | 1.1 |
| CB2(*h*) | | | | |
| | WIN55212 -2 | 1.4E-09 | 9.2E-09 | 0.7 |

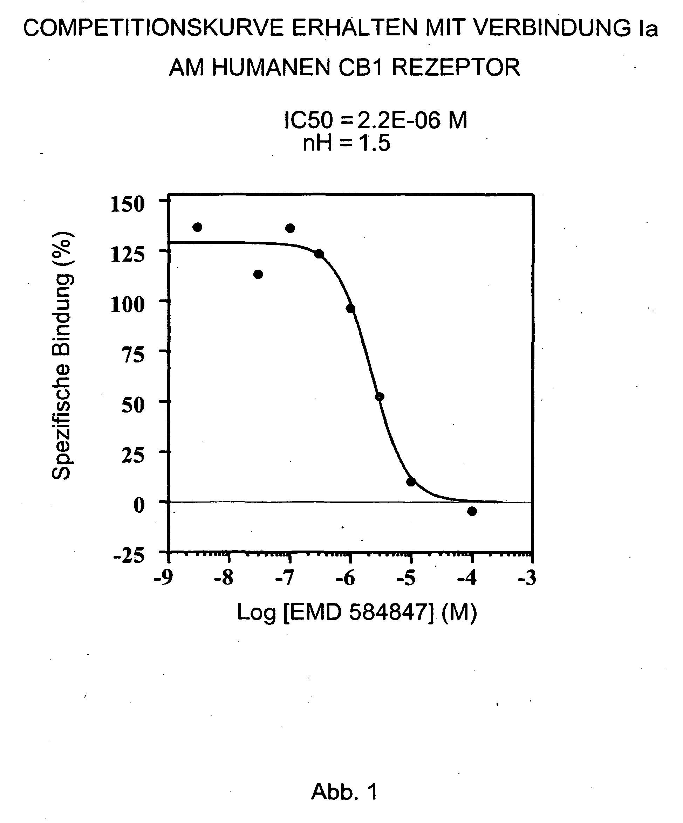

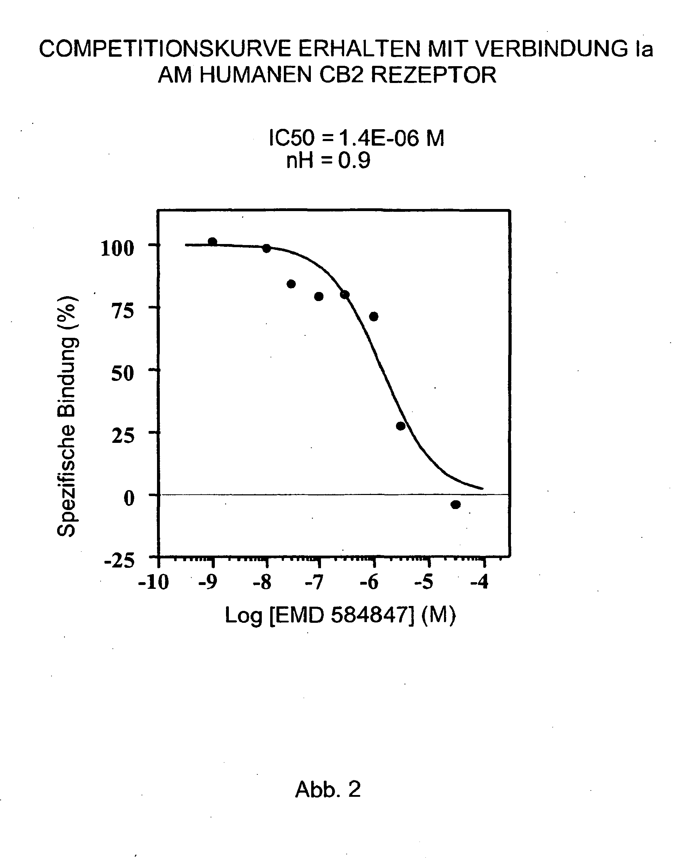

Die mit der Verbindung Ia erhaltenen Competitionskurven sind in den Abb. 1 und 2 dargestellt.

Die nachfolgenden Beispiele betreffen pharmazeutische Zubereitungen:

### Beispiel A: Injektionsgläser

Eine Lösung von 100 g eines Wirkstoffes der Formel I und 5 g Dinatriumhydrogenphosphat wird in 3 I zweifach destilliertem Wasser mit 2 N Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I, 9,38 g NaH₂PO₄ · 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 I auf und sterilisiert durch Bestrahlung. Diese Lösung kann in Form von Augentropfen verwendet werden.

### Beispiel D: Salbe

Man mischt 500 mg eines Wirkstoffes der Formel I mit 99,5 g Vaseline unter aseptischen Bedingungen.

### Beispiel E: Tabletten

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I in 60 I zweifach destilliertem Wasser wird steril filtriert, in Ampullen abgefüllt, unter sterilen Bedingungen lyophilisiert und steril verschlossen. Jede Ampulle enthält 10 mg Wirkstoff.

### Beispiel I: Inhalations-Spray

Man löst 14 g Wirkstoff der Formel I in 10 I isotonischer NaCl-Lösung und füllt die Lösung in handelsübliche Sprühgefäße mit Pump-Mechanismus. Die Lösung kann in Mund oder Nase gesprüht werden. Ein Sprühstoß
(etwa 0,1 ml) entspricht einer Dosis von etwa 0,14 mg.

## Patentansprüche

1. Verbindungen der Formel I 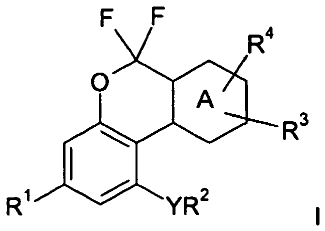 worin
A einen gesättigten oder ungesättigten carbocyclischen oder heterocyclischen Rest mit 6 Ringgliedern, worin bei gesättigten Resten eine oder mehrere CH₂-Gruppen durch CO ersetzt sein können,
R¹ R², R³, R⁴ unabhängig voneinander H, Hal, verzweigtes oder unverzweigtes Alkyl mit 1 bis 12 C-Atomen, worin auch eine oder mehrere H-Atome durch F, eine oder mehrere CH₂-Gruppen durch -C=C-, -C≡C-, C=O, -O-, -S-, -NH-, -NR¹-oder Phenylen und eine oder mehrere CH₃-Gruppen durch 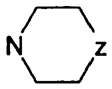
ersetzt sein können, worin z, CHR⁵, S, O, NR⁵ oder NR⁵ bedeutet,
Hal Fluor, Chlor, Brom oder Iod,
Y eine Einfachbindung, O, S oder NR⁵;
R⁵, H oder Alkyl
bedeuten,
sowie deren physiologisch unbedenkliche Salze und Solvate, Steroisomere und deren Mischungen in allen Verhältnissen.

2. Verbindungen nach Anspruch 1, worin R¹ Alkyl bedeutet.

3. Verbindungen nach Anspruch 1 oder 2, worin R³ und/oder R⁴ H, OH, Alkyl oder Alkoxy bedeutet.

4. Verbindungen nach Anspruch 1 bis 3 worin Y O bedeutet.

5. Verbindungen nach Anspruch 1 bis 4, worin R² H, Alkyl oder 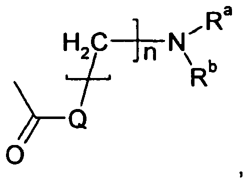 worin
n 1,2,3,4,5,6,7,8,9,10,11 oder 12
Q O, NH, N-Alkyl oder CH₂,
R^{a}, R^{b} unabhängig H oder Alkyl oder die Gruppe NR^{a}R^{b} zusammen auch 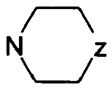 bedeutet, worin
z CH₂, S, O, NR¹ oder NH bedeutet.

6. Verbindungen nach Anspruch 5, worin Q O bedeutet.

7. Verbindungen nach Anspruch 5 oder 6, worin die Gruppe NR^{a}R^{b} 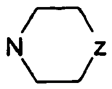 bedeutet.

8. Verbindungen nach Anspruch 1 mit den Formeln I1 bis 139 und la:

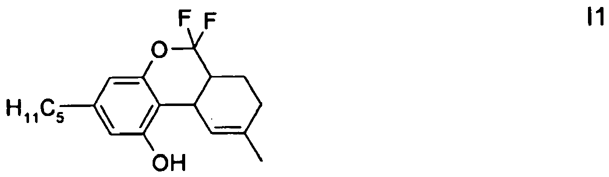

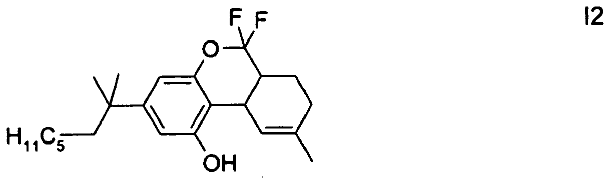

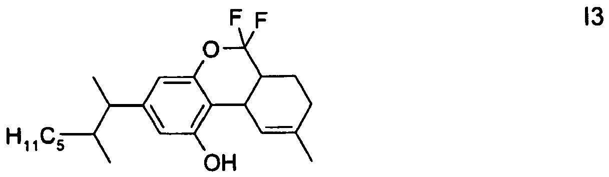

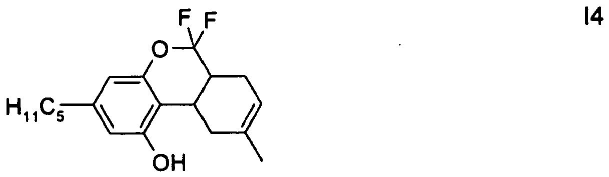

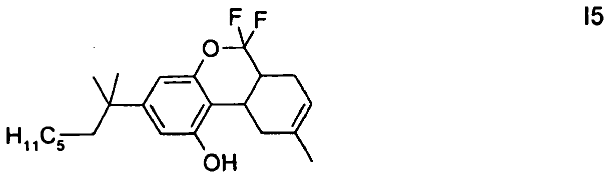

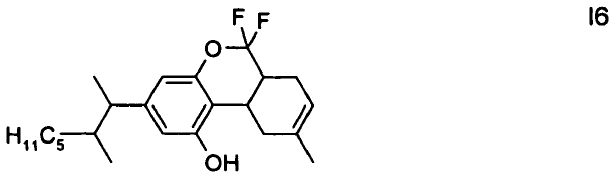

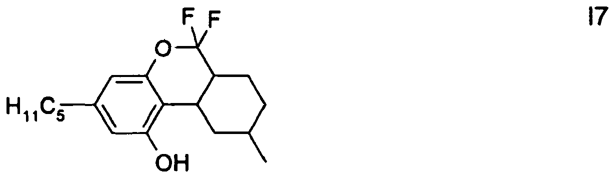

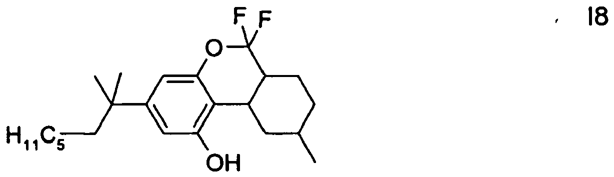

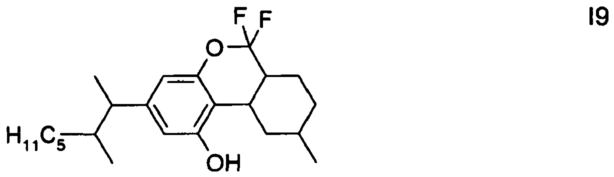

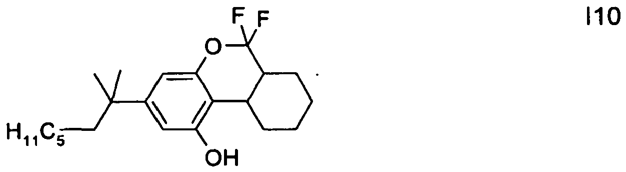

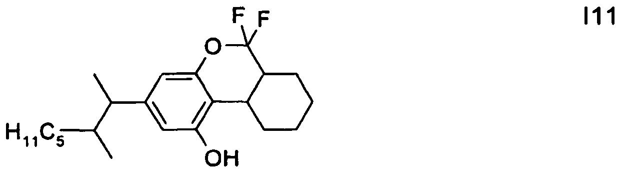

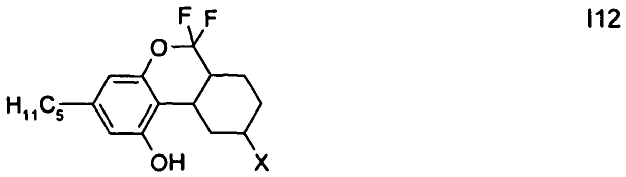

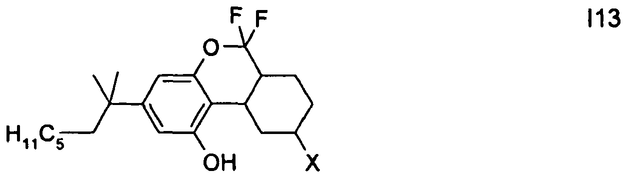

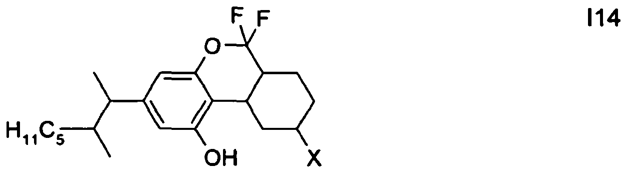

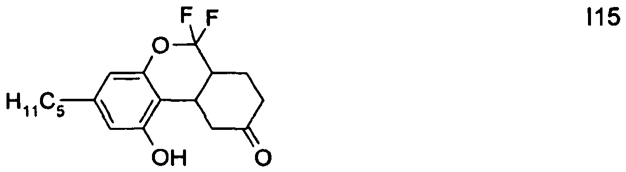

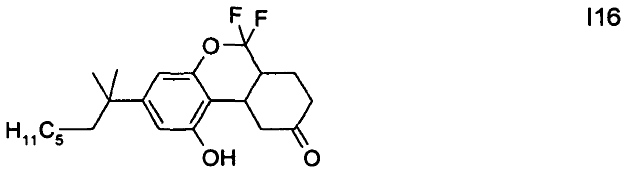

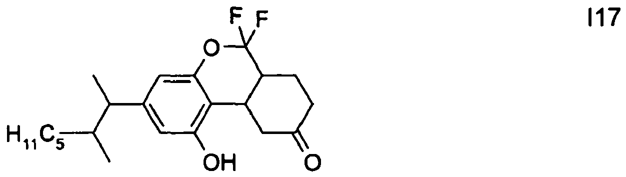

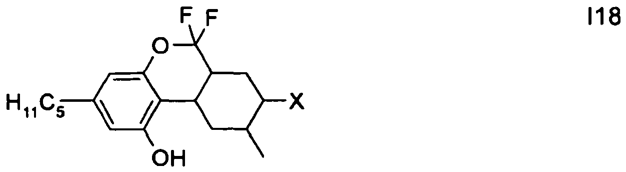

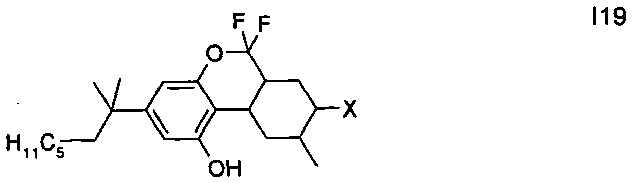

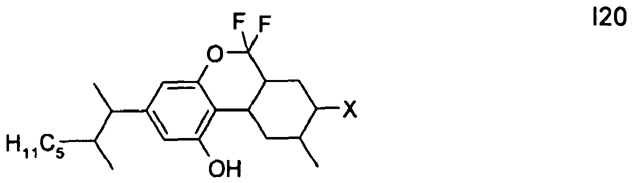

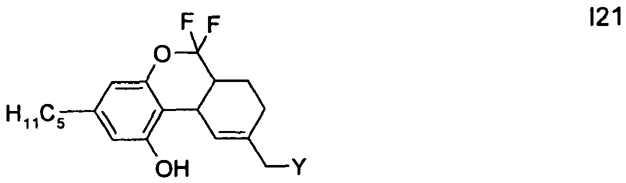

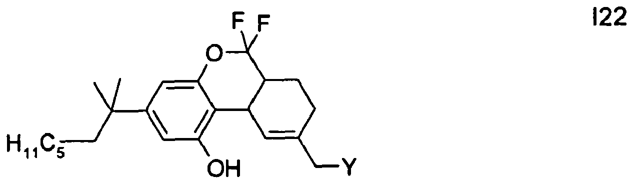

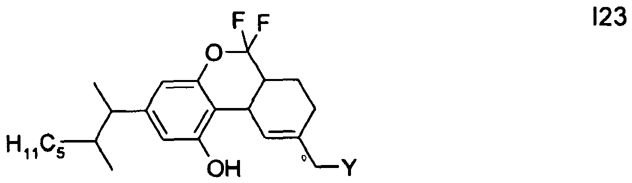

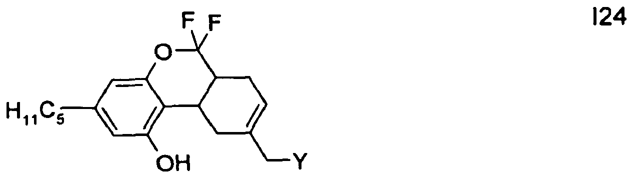

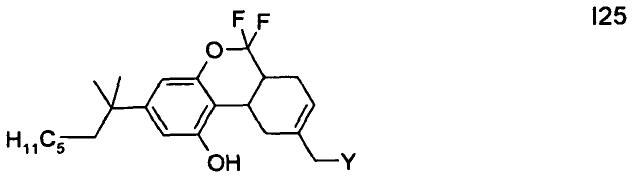

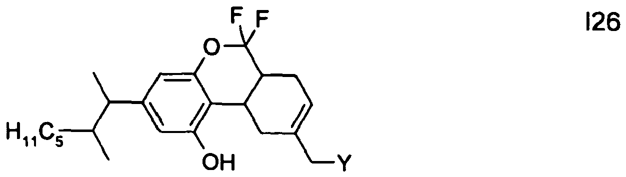

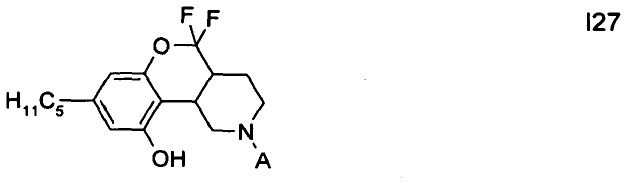

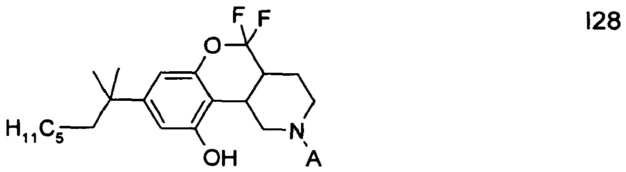

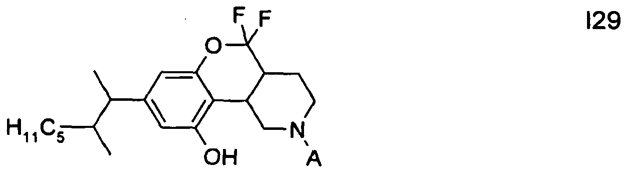

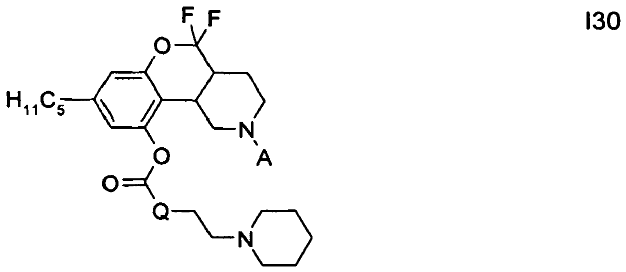

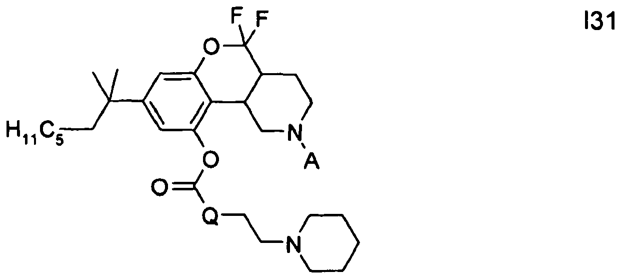

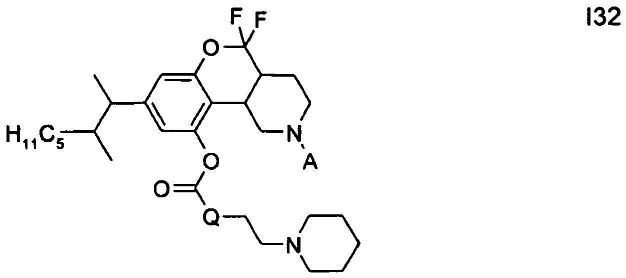

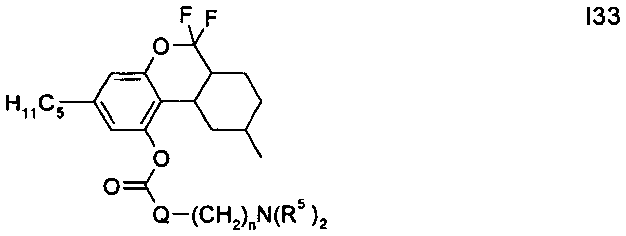

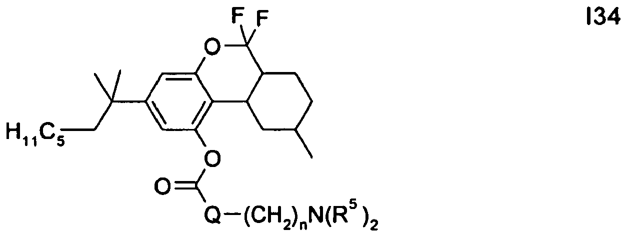

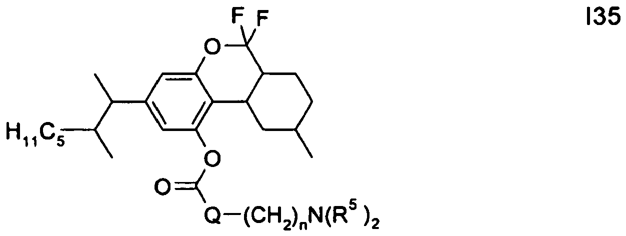

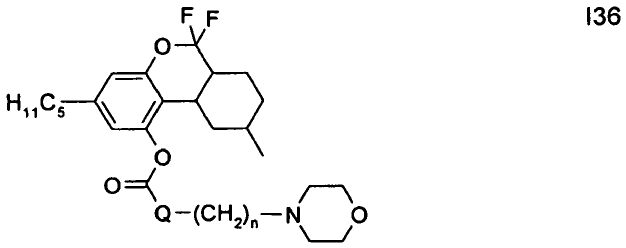

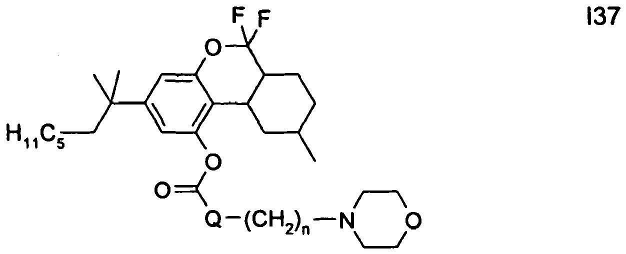

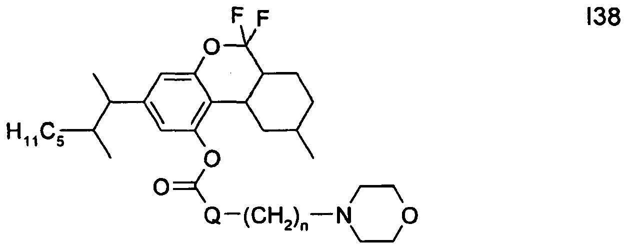

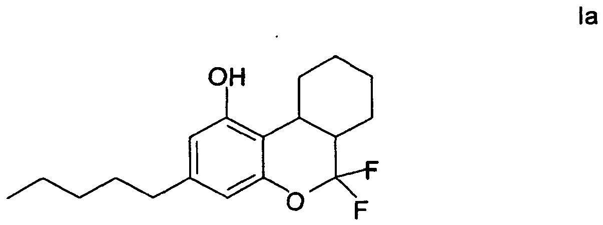

9. Verfahren zur Herstellung der Verbindungen der Formel I durch Umsetzung von Verbindungen der Formel II 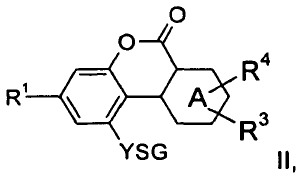 **gekennzeichnet durch** folgende Schritte:
a) Umsetzung der Verbindungen der Formel II mit aa) einem Thionierungsreagenz oder ab) mit einer Verbindung der Formel (Alkyl)₂Al-S-(CH₂)ₘ-S-Al(Alkyl)₂, worin Alkyl Methyl, Ethyl, n-Propyl, iso-propyl, n-Butyl, sek-Butyl oder tert-Butyl und m 2 oder 3 bedeuten;
b) Oxidative Fluorierung der in aa) oder ab) erhaltenen Verbindungen mit einem Oxidationsmittel, das Haloniumequivalente freisetzt, in Gegenwart einer Fluoridquelle, oder **durch** Umsetzung der in ab) erhaltenen Thionolactone mit Fluorierungsreagenzien, und nachfolgende Entschützung zu den Verbindungen der Formel I.

10. Verfahren zur Herstellung von Verbindungen der Formel I, **gekennzeichnet durch** folgende Schritte:
a) Umsetzung einer Verbindung der Formel III 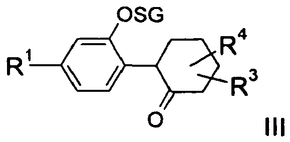 worin R¹, R³, und R⁴ die oben angegebene Bedeutung aufweisen, mit einer Verbindung der folgenden Formel 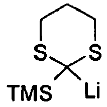
b) Oxidative Fluorierung der in a) erhaltenen Verbindungen mit einem Oxidationsmittel, das Haloniumequivalente freisetzt, in Gegenwart einer Fluoridquelle, und nachfolgende Entschützung zu den Verbindungen der Formel I.

11. Verbindungen der Formel I nach Anspruch 1 und ihre physiologisch unbedenklichen Salze oder Solvate als therapeutische Wirkstoffe.

12. Pharmazeutische Zubereitung, **gekennzeichnet durch** einen Gehalt an mindestens einer Verbindung der Formel I nach Anspruch 1 und/oder ihre physiologisch unbedenklichen Salze oder Solvate.

13. Verbindungen der Formel I nach Anspruch 1 und/oder ihre physiologisch unbedenklichen Salze oder Solvate zur Verwendung bei der Behandlung oder Prophylaxe von Psychosen, Angststörungen, Depression, Aprosexie, Gedächtnisstörungen, kognitive Störungen, Appetitlosigkeit, Obesitas, Sucht, Drogenabhängigkeit und neurologischen Störungen wie neurodegenerative Prozesse, Demenz, Dystonie, Muskelspasmen, Tremor, Epilepsie, Multiple Sklerose, traumatische Hirnverletzungen, Schlaganfall, Parkinson, Alzheimer, Huntington's Disease, Tourette-Syndrom, cerebrale Ischämie, cerebralem Apoplex, craniocerebralem Trauma, Rückenmarksverletzungen, neuroinflammatorische Krankheiten, cerebrale Arteriosklerose, virale Encephalitis, Krankheiten, die mit einer Demyelinisierung einhergehen, sowie zur Behandlung von Schmerzkrankheiten einschließlich neuropathischer Schmerzkrankheiten, und der Krankheiten septischer Schock, grüner Star, Krebs, Diabetes, Erbrechen, Übelkeit, Asthma, Atemwegserkrankungen, gastrointestinale Erkrankungen, Magengeschwür, Diarrhoe und kardiovaskuläre Krankheiten.

## Claims

1. Compounds of the formula I 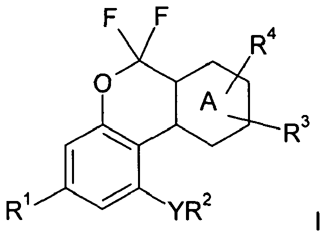 in which
A denotes a saturated or unsaturated carbocyclic or heterocyclic radical having 6 ring members, in which, in the case of saturated radicals, one or more CH₂ groups may be replaced by CO,
R¹, R², R³, R⁴, independently of one another, denote H, Hal, branched or unbranched alkyl having 1 to 12 C atoms, in which, in addition, one or more H atoms may be replaced by F, one or more CH₂ groups may be replaced by -C=C-, -C≡C-, C=O, -O-, -S-, -NH-, -NR¹- or phenylene, and one or more CH₃ groups may be replaced by 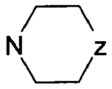 in which z denotes CHR⁵, S, O, NR⁵ or NR⁵,
Hal denotes fluorine, chlorine, bromine or iodine,
Y denotes a single bond, O, S or NR⁵,
R⁵ denotes H or alkyl,
and physiologically acceptable salts and solvates, stereoisomers thereof and mixtures thereof in all ratios.

2. Compounds according to Claim 1 in which R¹ denotes alkyl.

3. Compounds according to Claim 1 or 2 in which R³ and/or R⁴ denote H, OH, alkyl or alkoxy.

4. Compounds according to Claims 1 to 3 in which Y denotes O.

5. Compounds according to Claims 1 to 4 in which R² denotes H, alkyl or 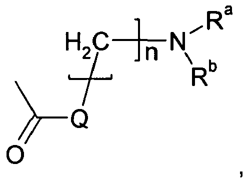 in which
n denotes 1,2,3,4,5,6,7,8,9,10,11 or 12
Q denotes O, NH, N-alkyl or CH₂,
R^{a}, R^{b} independently denote H or alkyl
or the NR^{a}R^{b} group together also denotes 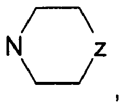 in which
z denotes CH₂, S, O, NR¹ or NH.

6. Compounds according to Claim 5 in which Q denotes O.

7. Compounds according to Claim 5 or 6 in which the NR^{a}R^{b} group denotes

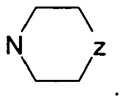

8. Compounds according to Claim 1 of the formulae I1 to I39 and la:

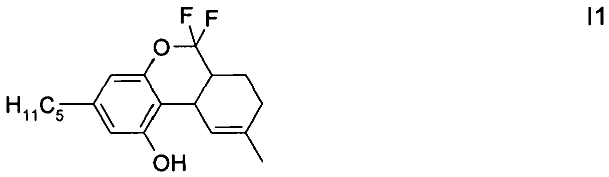

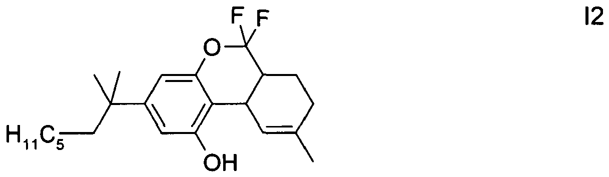

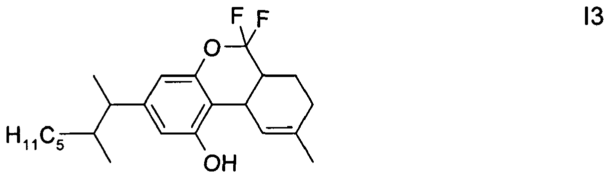

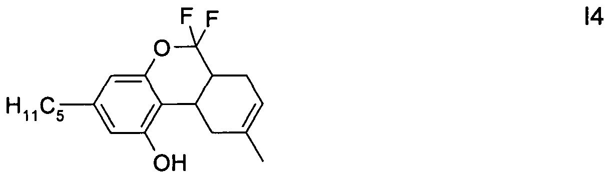

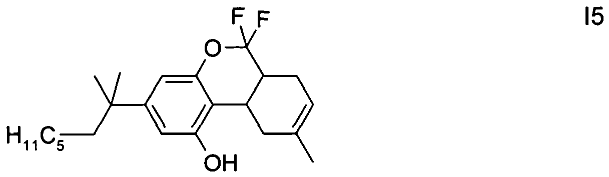

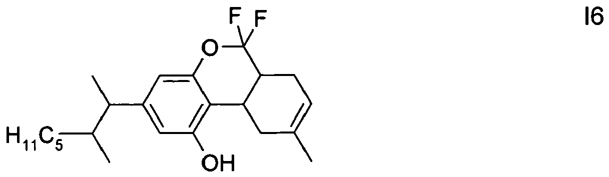

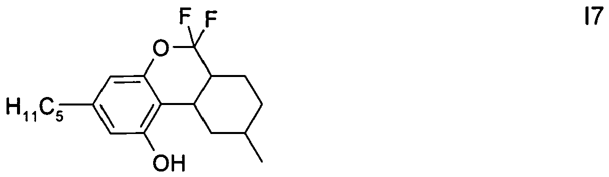

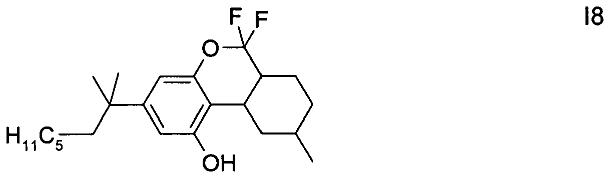

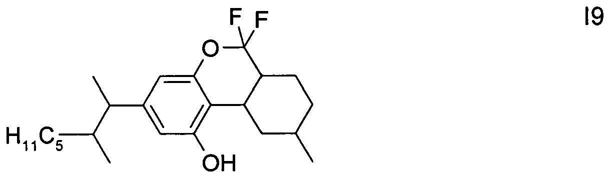

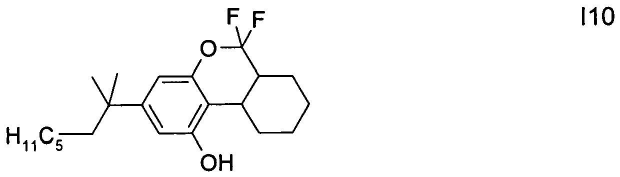

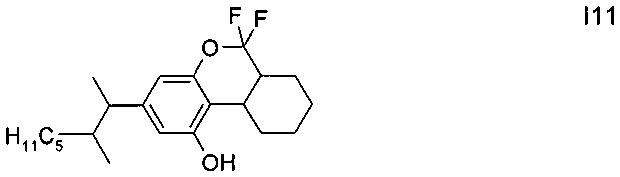

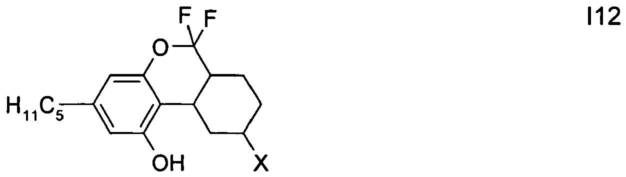

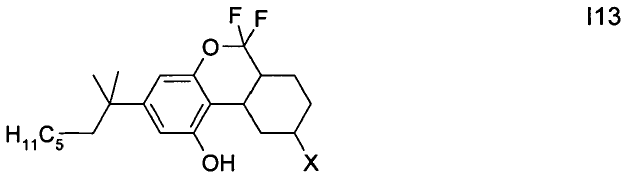

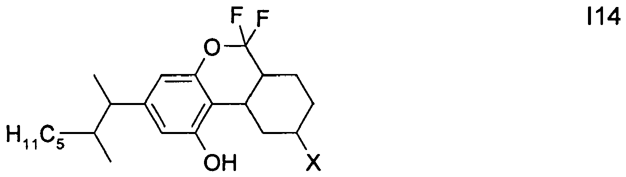

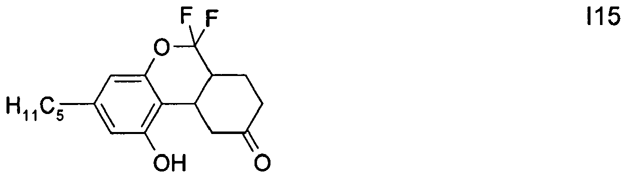

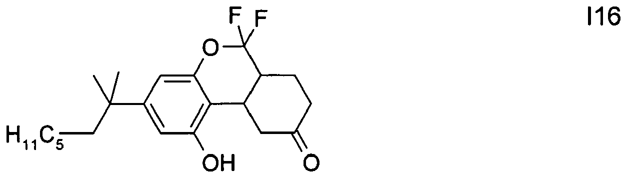

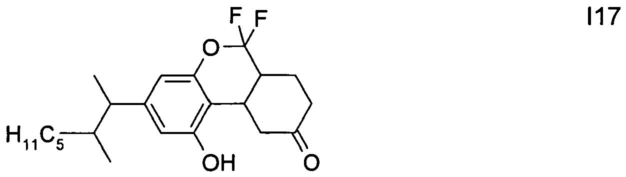

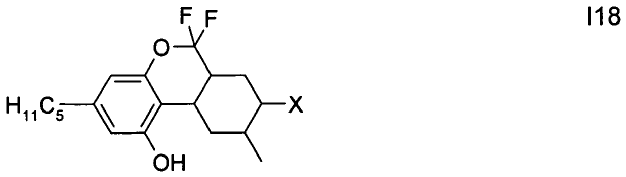

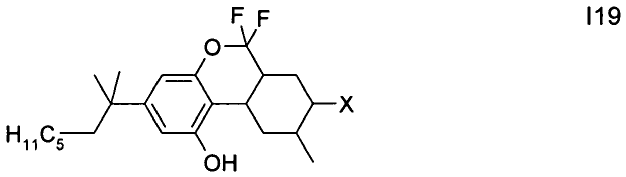

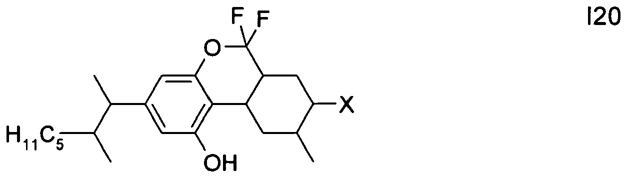

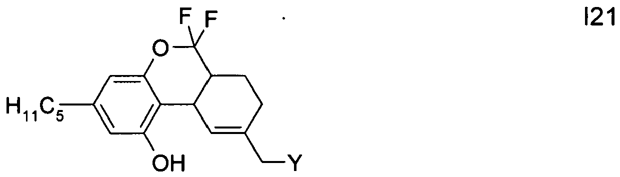

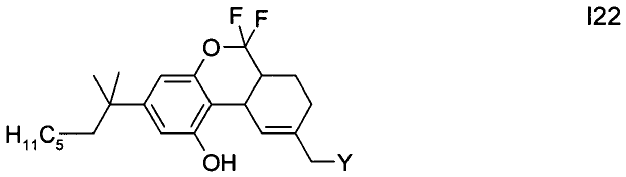

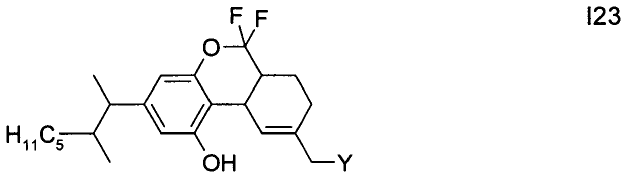

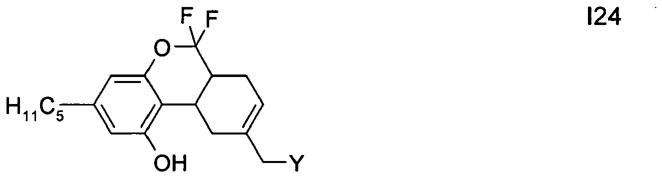

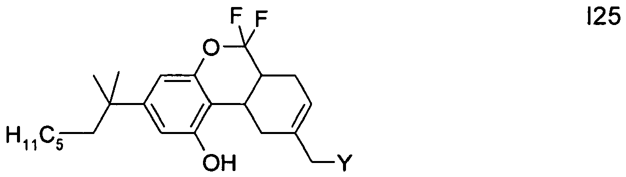

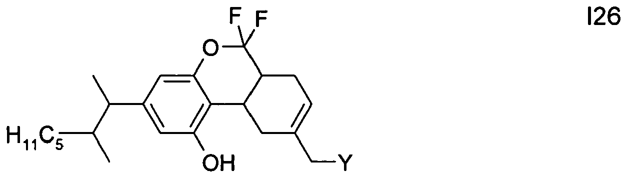

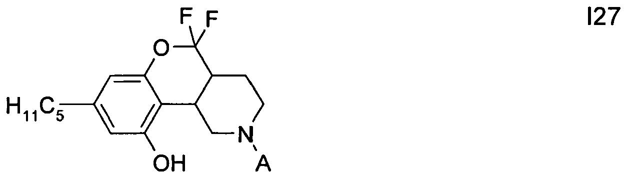

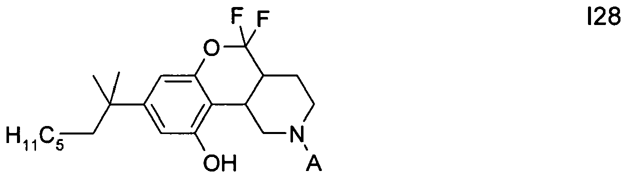

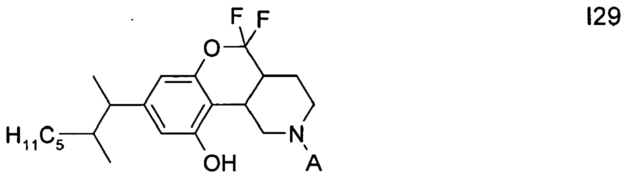

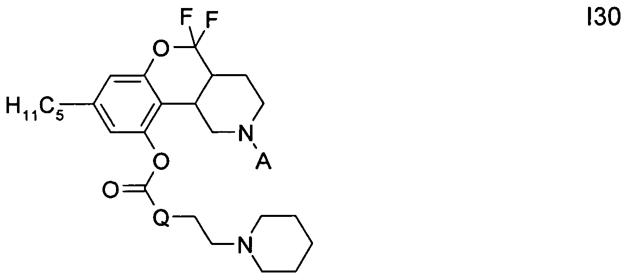

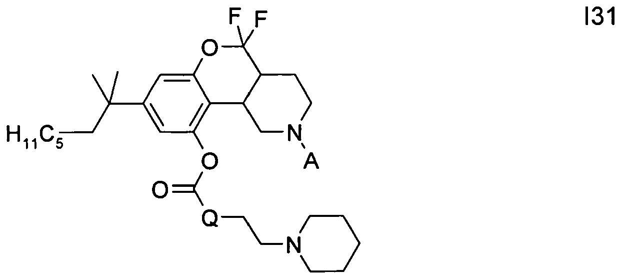

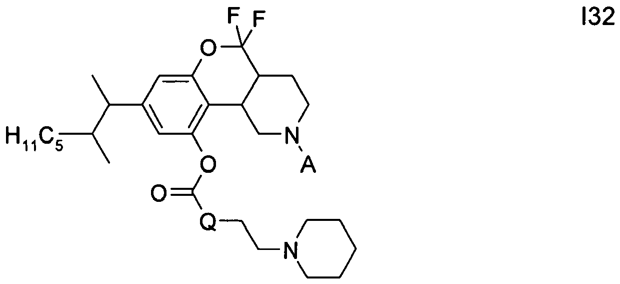

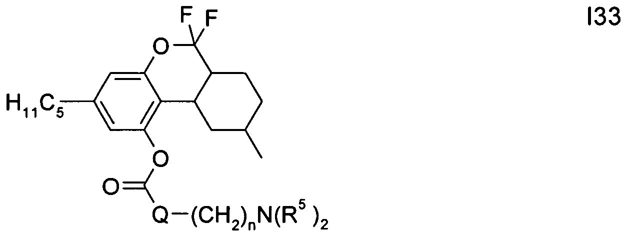

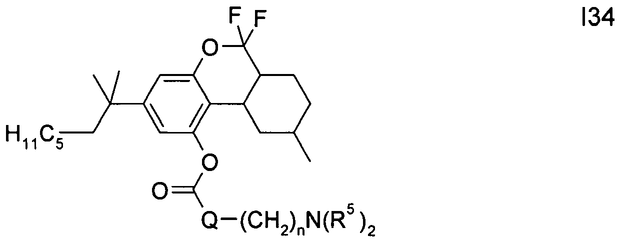

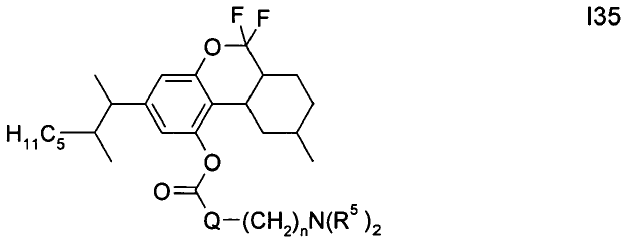

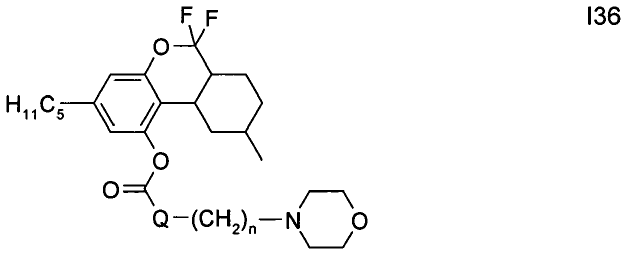

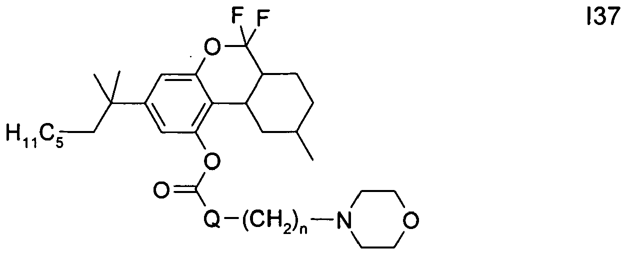

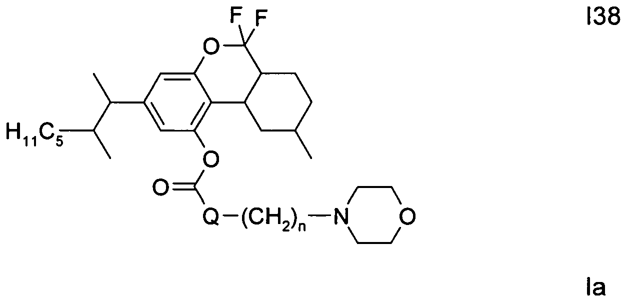

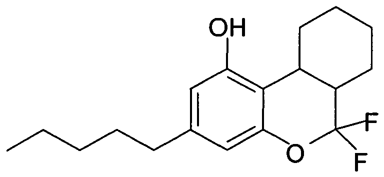

9. Process for the preparation of the compounds of the formula I by reaction of compounds of the formula II 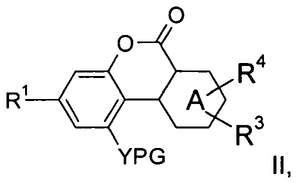 **characterised by** the following steps:
a) reaction of the compounds of the formula II with aa) a thionation reagent or ab) with a compound of the formula (alkyl)₂Al-S-(CH₂)ₘ-SAl(alkyl)₂, in which alkyl denotes methyl, ethyl, n-propyl, isopropyl, n-butyl, sec-butyl or tert-butyl, and m denotes 2 or 3;
b) oxidative fluorination of the compounds obtained in aa) or ab) using an oxidant which liberates halonium equivalents, in the presence of a fluoride source, or by reaction of the thionolactones obtained in ab) with fluorinating reagents, and subsequent deprotection to give the compounds of the formula I.

10. Process for the preparation of compounds of the formula I, **characterised by** the following steps:
a) reaction of a compound of the formula III 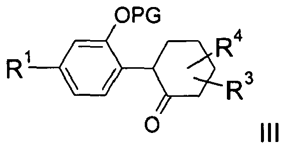 in which R¹, R³ and R⁴ have the meaning indicated above, with a compound of the following formula: 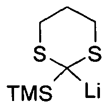
b) oxidative fluorination of the compounds obtained in a) using an oxidant which liberates halonium equivalents, in the presence of a fluoride source, and subsequent deprotection to give the compounds of the formula I.

11. Compounds of the formula I according to Claim 1 and physiologically acceptable salts or solvates thereof as therapeutic active compounds.

12. Pharmaceutical composition, **characterised by** a content of at least one compound of the formula I according to Claim 1 and/or physiologically acceptable salts or solvates thereof.

13. Compounds of the formula I according to Claim 1 and/or physiologically acceptable salts or solvates thereof for use in the treatment or prophylaxis of psychoses, anxiety disorders, depression, aprosexia, memory disorders, cognitive disorders, loss of appetite, obesity, addiction, drug dependence and neurological disorders, such as neurodegenerative processes, dementia, dystonia, muscle spasms, tremor, epilepsy, multiple sclerosis, traumatic brain injuries, strokes, Parkinson's, Alzheimer's, Huntington's disease, Tourette's syndrome, cerebral ischaemia, cerebral apoplexy, craniocerebral trauma, spinal cord injuries, neuroinflammatory diseases, cerebral arteriosclerosis, viral encephalitis, diseases associated with demyelinisation, and for the treatment of pain diseases, including neuropathic pain diseases, and the diseases septic shock, glaucoma, cancer, diabetes, vomiting, nausea, asthma, respiratory tract diseases, gastrointestinal diseases, gastric ulcer, diarrhoea and cardiovascular diseases.

## Revendications

1. Composés de formule I 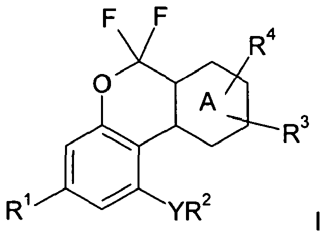 dans laquelle
A désigne un radical carbocyclique ou hétérocyclique saturé ou insaturé ayant 6 chaînons, dans lequel, dans le cas de radicaux saturés, un ou plusieurs groupements CH₂ peuvent être remplacés par CO,
R¹, R², R³, R⁴, désignent, indépendamment les uns des autres, H, Hal, alkyle ramifié ou non ramifié ayant de 1 à 12 atomes de C, où, de plus, un ou plusieurs atomes de H peuvent être remplacés par F, un ou plusieurs groupements CH₂ peuvent être remplacés par -C=C-, -C≡C-, C=O, -O-, -S-, -NH-, -NR¹- ou phénylène, et un ou plusieurs groupements CH₃ peuvent être remplacés par 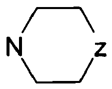 où z désigne CHR⁵, S, O, NR⁵ ou NR⁵,
Hal désigne fluor, chlore, brome ou iode,
Y désigne une liaison simple, O, S ou NR⁵,
R⁵ désigne H ou alkyle,
et les sels et solvats physiologiquement acceptables, les stéréoisomères de ceux-ci et des mélanges de ceux-ci en toutes proportions.

2. Composés selon la revendication 1, dans lesquels R¹ désigne alkyle.

3. Composés selon la revendication 1 ou 2, dans lesquels R³ et/ou R⁴ désignent H, OH, alkyle ou alcoxy.

4. Composés selon les revendications 1 à 3, dans lesquels Y désigne O.

5. Composés selon les revendications 1 à 4, dans lesquels R² désigne H, alkyle ou 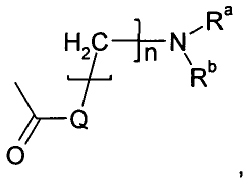 où
n désigne 1,2,3,4,5,6,7,8,9,10,11 ou 12
Q désigne O, NH, N-alkyle ou CH₂,
R^{a}, R^{b} désignent indépendamment H ou alkyle
ou le groupement NR^{a}R^{b} désigne aussi ensemble 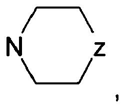 où
z désigne CH₂, S, O, NR¹ ou NH.

6. Composés selon la revendication 5, dans lesquels Q désigne O.

7. Composés selon la revendication 5 ou 6, dans lesquels le groupement NR^{a}R^{b} désigne

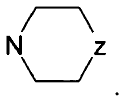

8. Composés selon la revendication 1, de formules I1 à 139 et la :

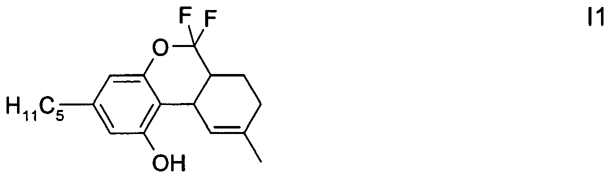

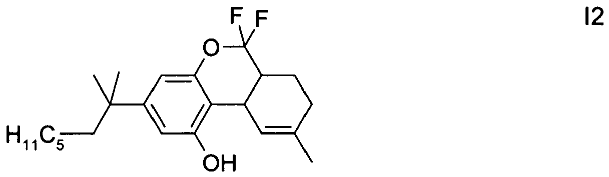

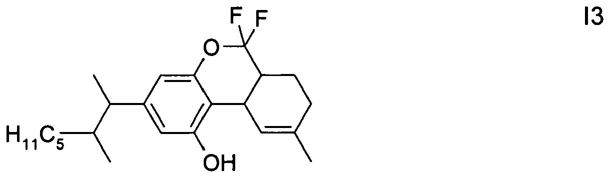

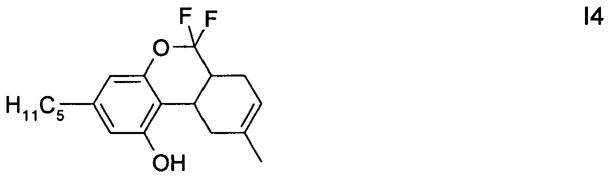

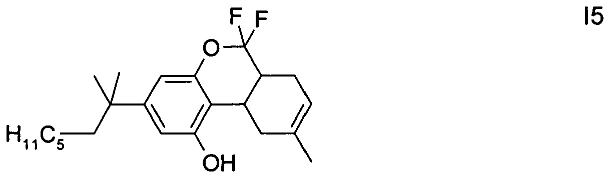

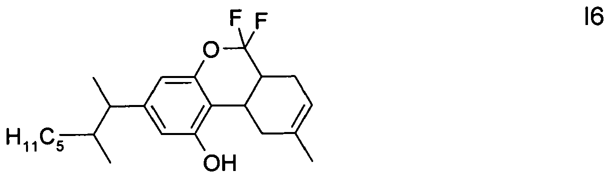

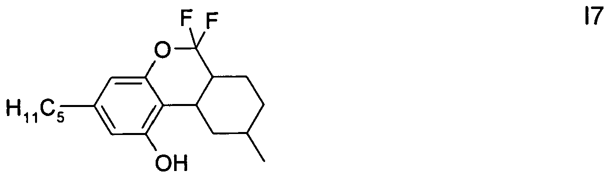

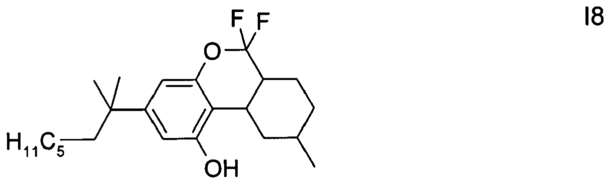

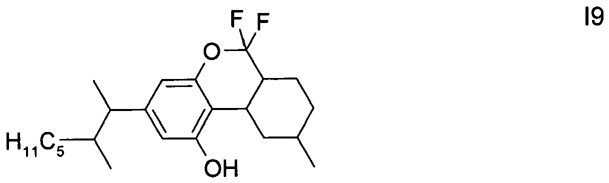

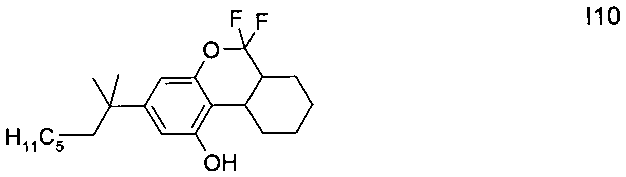

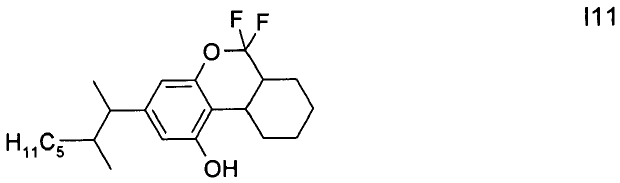

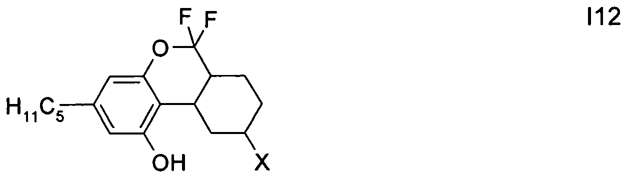

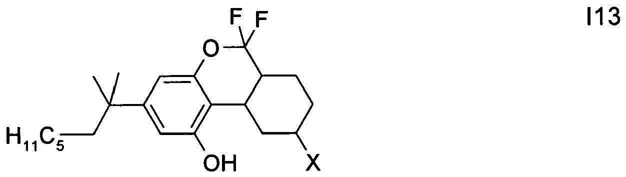

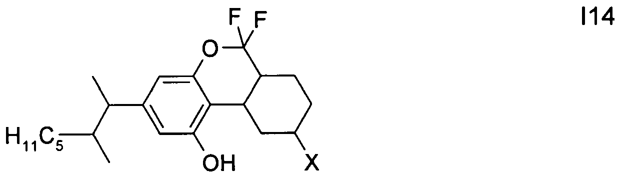

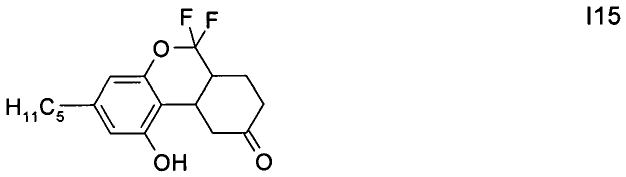

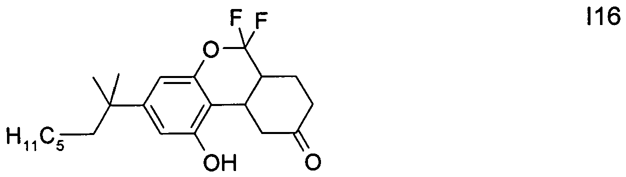

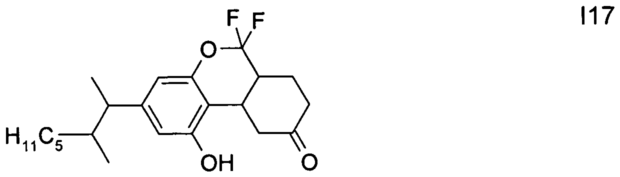

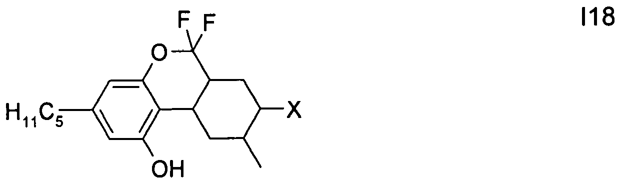

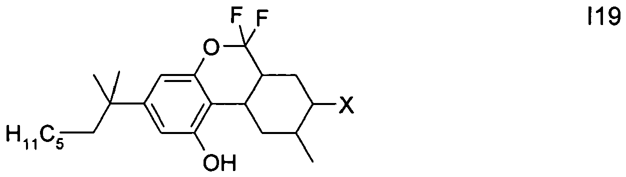

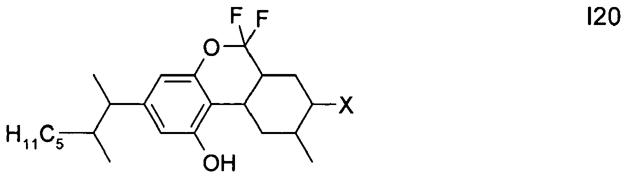

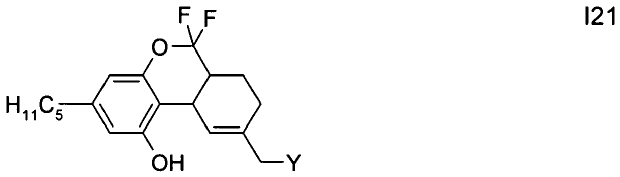

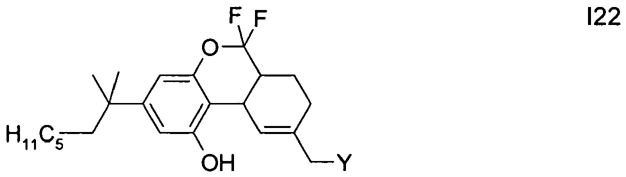

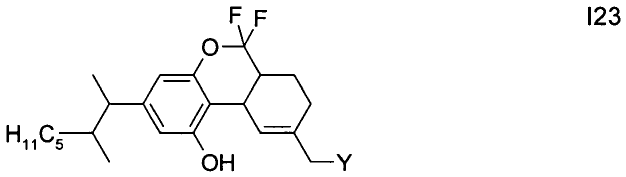

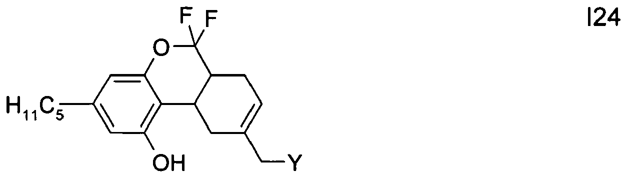

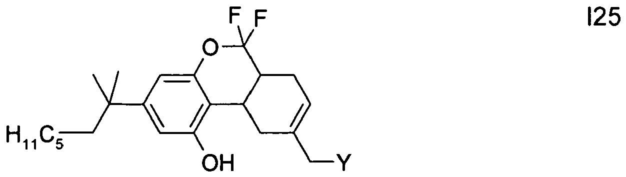

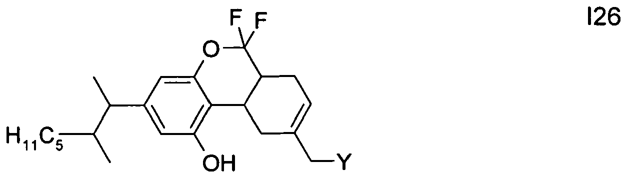

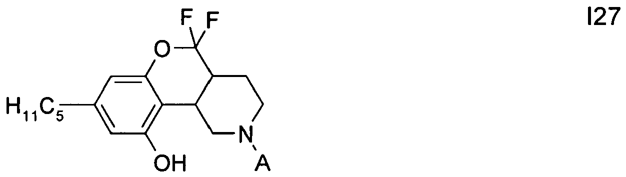

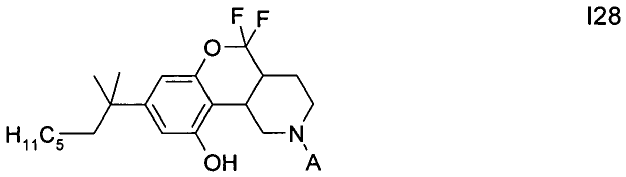

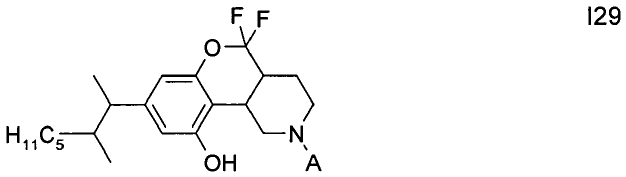

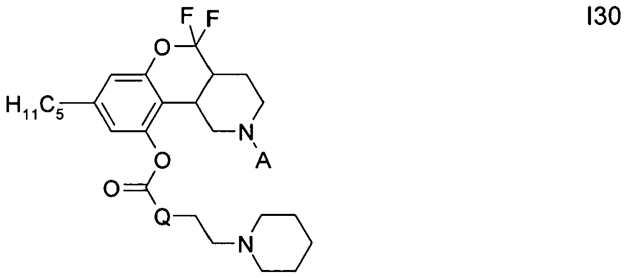

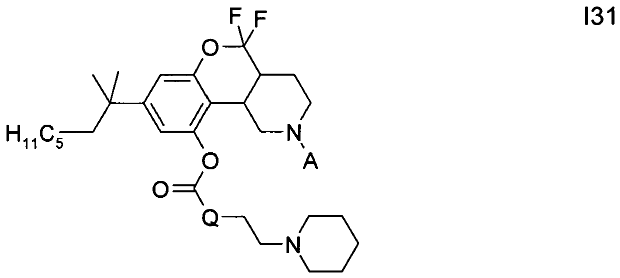

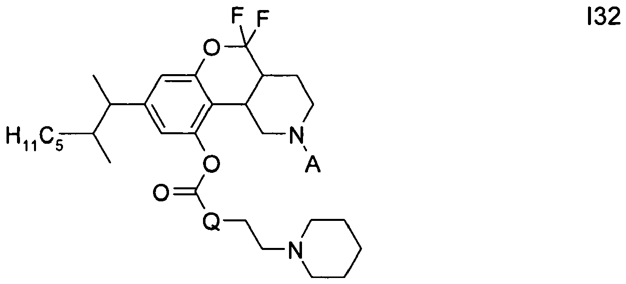

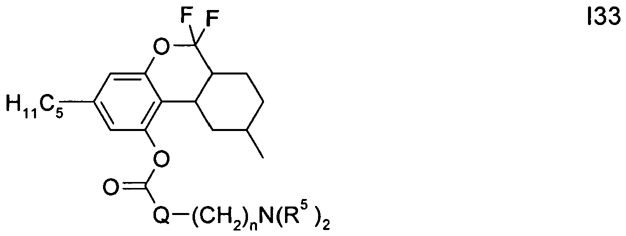

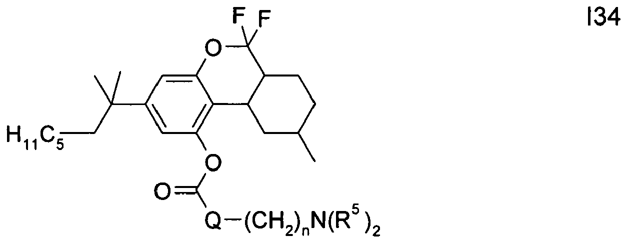

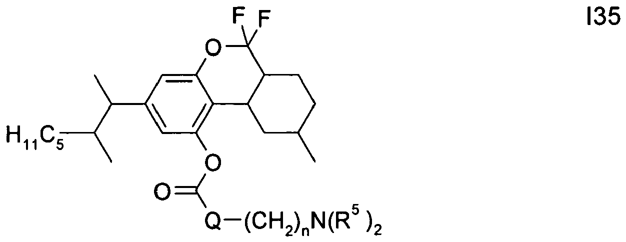

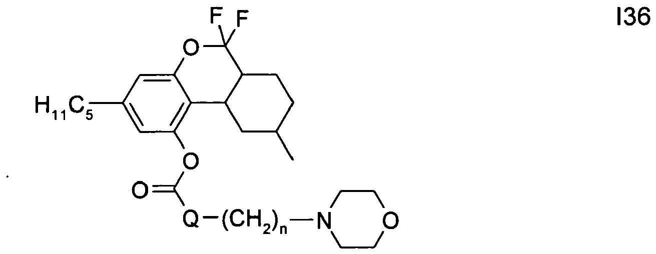

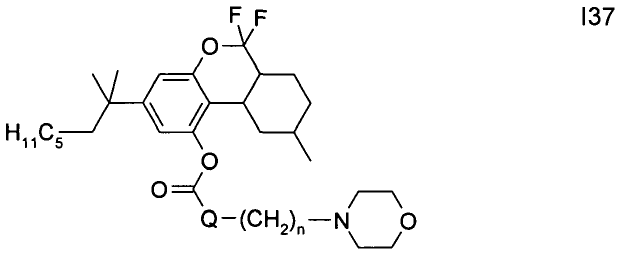

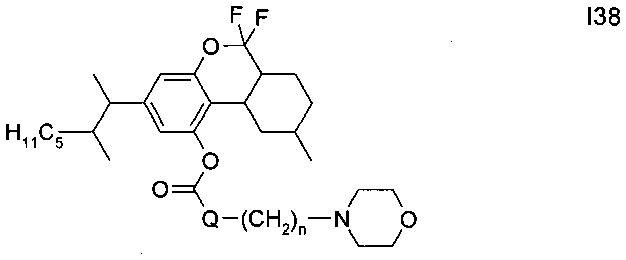

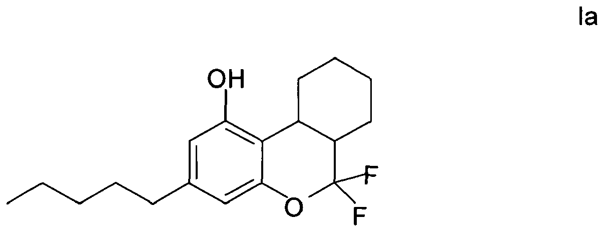

9. Procédé de préparation des composés de formule I par la réaction de composés de formule II 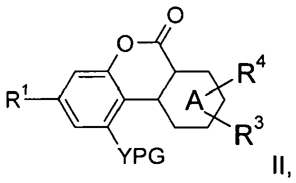 **caractérisé par** les étapes suivantes :
a) la réaction des composés de formule II avec aa) un réactif de thionation ou ab) avec un composé de formule (alkyl)₂Al-S-(CH₂)ₘ-S-Al(alkyl)₂, où alkyle désigne méthyle, éthyle, n-propyle, isopropyle, n-butyle, sec-butyle ou tertio-butyle, et m désigne 2 ou 3;
b) la fluoration oxydative des composés obtenus dans aa) ou ab) à l'aide d'un oxydant qui libère des équivalents halonium, en présence d'une source de fluorure, ou par réaction des thionolactones obtenues dans ab) avec des réactifs de fluoration, puis une déprotection pour conduire aux composés de formule I.

10. Procédé de préparation des composés de formule I, **caractérisé par** les étapes suivantes :
a) la réaction d'un composé de formule III 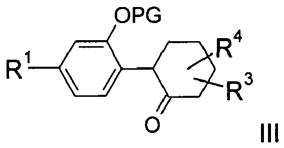 dans laquelle R¹, R³ et R⁴ revêtent les significations indiquées ci-dessus,
avec un composé de formule suivante : 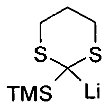
b) la fluoration oxydative des composés obtenus dans a) à l'aide d'un oxydant qui libère des équivalents halonium, en présence d'une source de fluorure, puis une déprotection pour conduire aux composés de formule I.

11. Composés de formule I selon la revendication 1, et les sels ou solvats physiologiquement acceptables de ceux-ci, comme composés actifs thérapeutiques.

12. Composition pharmaceutique, **caractérisée par** une teneur en au moins un composé de formule I selon la revendication 1 et/ou en sels ou solvats physiologiquement acceptables de celui-ci.

13. Composés de formule I selon la revendication 1 et/ou les sels ou solvats physiologiquement acceptables de ceux-ci, pour une utilisation dans le traitement ou la prophylaxie de psychoses, de troubles anxieux, de la dépression, de l'aprosexie, de troubles de la mémoire, de troubles cognitifs, de la perte d'appétit, de l'obésité, des attachements maladifs, de la pharmacodépendance et de troubles neurologiques, tels que les processus neurodégénératifs, la démence, la dystonie, les spasmes musculaires, les tremblements, l'épilepsie, la sclérose en plaques, les lésions traumatiques du cerveau, , la maladie de Parkinson, la maladie d'Alzheimer, la chorée de Huntington, le syndrome de Tourette, l'ischémie cérébrale, l'apoplexie cérébrale, les traumatismes cranio-cérébraux, les lésions de la moelle épinière, les maladies neuroinflammatoires, l'artériosclérose cérébrale, l'encéphalite virale, les maladies associées à une démyélinisation, et pour le traitement de maladies liées à la douleur, y compris les maladies de douleur neuropathique, et les maladies de choc septique, de glaucome, de cancer, de diabète, de vomissements, de nausée, d'asthme, les maladies du tractus respiratoire, les maladies gastrointestinales, l'ulcère gastrique, la diarrhée et les maladies cardiovasculaires.
